# EUROPEAN PATENT APPLICATION

(11) **EP 2 119 442 A1**
(43) Date of publication of application: **18.11.2009**
(21) Application number: 07860228.1
(22) Date of filing: 26.12.2007
(51) Int. Cl.: A61K 31/436, A61K 47/32, A61K 47/34, A61K 47/38

(54) **TACROLIMUS SUSTAINED RELEASE PHARMACEUTICAL COMPOSITION**

(30) Priority: 28.12.2006 US 877355 P
(71) Applicant: Astellas Pharma Inc., Tokyo 103-8411 (JP)
(72) Inventor: KONDO, Hiromu, Chuo-ku Tokyo 103-8411 (JP); KOJIMA, Hiroyuki, Chuo-ku Tokyo 103-8411 (JP); YOSHIHARA, Keiichi, Chuo-ku Tokyo 103-8411 (JP); TAKETANI, Yuko, Chuo-ku Tokyo 103-8411 (JP); ISHII, Takuya, Chuo-ku Tokyo 103-8411 (JP)
(74) Representative: Gille Hrabal Struck Neidlein Prop Roos
(86) International application number: PCT/JP2007/074999
(87) International publication number: WO 2008/084698

(57) **Abstract**

A sustained release pharmaceutical composition for tacrolimus, comprising a solid dispersion containing tacrolimus or a pharmaceutically acceptable salt thereof, and a carrier for a sustained release pharmaceutical composition, wherein a dissolution rate of tacrolimus after 4 hours from the beginning of a dissolution test is less than 35%, is disclosed.

## Description

### TECHNICAL FIELD

The present invention relates to a sustained release pharmaceutical composition for tacrolimus, characterized by comprising a solid dispersion containing tacrolimus or a pharmaceutically acceptable salt thereof, and a carrier for a sustained release pharmaceutical composition, wherein a dissolution rate of tacrolimus after 4 hours from the beginning of dissolution is less than 35%.

### BACKGROUND ART

Several techniques for sustained release pharmaceutical compositions for tacrolimus have been reported. For example, patent reference 1 discloses a pharmaceutical composition in which 63.2% of tacrolimus is released for 0.7 to 15 hours. This technique is related to a tequnique achieving a good oral absorption of tacrolimus, a reduction of an absorption variability, and the sufficient maintenance of pharmacological effects of tacrolimus. However, patent reference 1 does not disclose nor suggest a sustained release pharmaceutical composition for tacrolimus which reduces food effects, that is, which reduces the change of pharmacokinetic parameters (PK parameters) by eating a meal and lowers a peak/trough ratio (hereinafter referred to as PT ratio) of a blood concentration as an index of a safety margin.

Patent reference 2 discloses a technique in which a decreased bioavailability based on CYP metabolism in the gastrointestinal tract is avoided by a composition prepared by coating a melted composition of tacrolimus with an enteric coating material. Since the solubility of an enteric coating material selected in this technique is affected by a pH in the gastrointestinal tract, there is an apprehension that the drug may be released at an inconstant site of the gastrointestinal tract to cause the variation of a blood concentration. This composition affected by such a factor of a living body is unsuitable as a pharmaceutical formulation for reducing food effects or improving the safety profile thereof. Further, patent reference 2 does not disclose nor suggest concrete techniques for reducing food effects, lowering the PT ratio, and/or improving the safety profile.

As described above, several sustained release pharmaceutical compositions containing tacrolimus are known, but a sustained release pharmaceutical composition for tacrolimus capable of reducing food effects and improving the safety profile is unknown. Further, a use of tacrolimus or a pharmaceutically acceptable salt thereof for the manufacture of a sustained release pharmaceutical composition for tacrolimus capable of avoiding food effects, and a use of tacrolimus or a pharmaceutically acceptable salt thereof for the manufacture of a sustained release pharmaceutical compositions for tacrolimus capable of improving the safety profile, are unknown.

[patent reference 1] International Publication WO 99/49863 [patent reference 2] International Publication WO 2005/020993

### DISCLOSURE OF INVENTION

### PROBLEMS TO BE SOLVED BY THE INVENTION

An object of the present invention is to provide a sustained release pharmaceutical composition for tacrolimus capable of inhibiting the change of PK parameters by the intake of food and obtaining a blood concentration profile showing a low PT ratio.

The present inventors found that, when a sustained release pharmaceutical composition for tacrolimus in which the release of tacrolimus is controlled under a certain level is orally administered to dogs, the change of PK parameters by food intake was significantly inhibited, the PT ratio was significantly lowered, and the safety profile was improved, and thus, the present invention was completed.

### MEANS FOR SOLVING THE PROBLEMS

The present invention relates to
1. a sustained release pharmaceutical composition for tacrolimus, comprising a solid dispersion containing tacrolimus or a pharmaceutically acceptable salt thereof, and a carrier for a sustained release pharmaceutical composition, wherein a dissolution rate of tacrolimus after 4 hours from the beginning of a dissolution test is less than 35%;
2. the sustained release pharmaceutical composition for tacrolimus as described in 1, wherein a ratio of a maximum blood tacrolimus concentration (Cmax) when administered after eating a meal to a maximum blood tacrolimus concentration when administered in a fasted state is 0.3 or more, and/or a ratio of an area under a blood tacrolimus concentration versus time curve (AUC) when administered after eating a meal to an area under a blood tacrolimus concentration versus time curve when administered in a fasted state is 0.5 or more;
3. the sustained release pharmaceutical composition for tacrolimus as described in 1, wherein a ratio of a maximum blood tacrolimus concentration (Cmax) when administered after eating a meal to a maximum blood tacrolimus concentration when administered in a fasted state is 0.7 or more, and/or a ratio of an area under a blood tacrolimus concentration versus time curve (AUC) when administered after eating a meal to an area under a blood tacrolimus concentration versus time curve when administered in a fasted state is 0.8 or more;
4. the sustained release pharmaceutical composition for tacrolimus as described in 1, wherein a ratio of a maximum blood tacrolimus concentration (Cmax) to a blood tacrolimus concentration after approximately 8 hours from oral administration of tacrolimus (C8h) is 5 or less;
5. the sustained release pharmaceutical composition for tacrolimus as described in 1, wherein a ratio of a maximum blood tacrolimus concentration (Cmax) to a blood tacrolimus concentration after approximately 24 hours from oral administration of tacrolimus (Cmin) is 3 or less;
6. the sustained release pharmaceutical composition for tacrolimus as described in 1, which is selected from the group consisting of a hydrogel-forming formulation, an osmotic pump type formulation, a gel formulation in which a plurality of gums is combined, a multi-layered tablet formulation consisting of a drug core and a release-controlling layer which are geometrically arranged, a formulation utilizing a swelling polymer, a matrix formulation utilizing a water-soluble polymer, and a sustained release formulation with a coating membrane;
7. the sustained release pharmaceutical composition for tacrolimus as described in 6, wherein the hydrogel-forming formulation comprises a hydrophilic base and a hydrogel-forming polymer;
8. a use of tacrolimus or a pharmaceutically acceptable salt thereof for the manufacture of a sustained release pharmaceutical composition for tacrolimus, wherein a dissolution rate of tacrolimus after 4 hours from the beginning of a dissolution test is less than 35%, and an effect of a meal on tacrolimus can be avoided;
9. the use of tacrolimus or a pharmaceutically acceptable salt thereof as described in 8, wherein a ratio of a maximum blood tacrolimus concentration (Cmax) when administered after eating a meal to a maximum blood tacrolimus concentration when administered in a fasted state is 0.3 or more, and/or a ratio of an area under a blood tacrolimus concentration versus time curve (AUC) when administered after eating a meal to an area under a blood tacrolimus concentration versus time curve when administered in a fasted state is 0.5 or more;
10. the use of tacrolimus or a pharmaceutically acceptable salt thereof as described in 8, wherein a ratio of a maximum blood tacrolimus concentration (Cmax) when administered after eating a meal to a maximum blood tacrolimus concentration when administered in a fasted state is 0.7 or more, and/or a ratio of an area under a blood tacrolimus concentration versus time curve (AUC) when administered after eating a meal to an area under a blood tacrolimus concentration versus time curve when administered in a fasted state is 0.8 or more;
11. the use of tacrolimus or a pharmaceutically acceptable salt thereof as described in 8, wherein the sustained release pharmaceutical composition for tacrolimus comprises a hydrophilic base and a hydrogel-forming polymer;
12. a use of tacrolimus or a pharmaceutically acceptable salt thereof for the manufacture of a sustained release pharmaceutical composition for tacrolimus, wherein a dissolution rate of tacrolimus after 4 hours from the beginning of a dissolution test is less than 35%, and the safety profile of tacrolimus can be improved;
13. the use of tacrolimus or a pharmaceutically acceptable salt thereof as described in 12, wherein a ratio of a maximum blood tacrolimus concentration (Cmax) to a blood tacrolimus concentration after approximately 8 hours from oral administration of tacrolimus (C8h) is 5 or less;
14. the use of tacrolimus or a pharmaceutically acceptable salt thereof as described in 12, wherein a ratio of a maximum blood tacrolimus concentration (Cmax) to a blood tacrolimus concentration after approximately 24 hours from oral administration of tacrolimus (Cmin) is 3 or less;
15. the use of tacrolimus or a pharmaceutically acceptable salt thereof as described in 12, wherein the sustained release pharmaceutical composition for tacrolimus comprises a hydrophilic base and a hydrogel-forming polymer;
16. a method of regulating a ratio of a maximum blood tacrolimus concentration (Cmax) when administered after eating a meal to a maximum blood tacrolimus concentration when administered in a fasted state to 0.3 or more, and/or a ratio of an area under a blood tacrolimus concentration versus time curve (AUC) when administered after eating a meal to an area under a blood tacrolimus concentration versus time curve when administered in a fasted state to 0.5 or more, by dissolving tacrolimus contained in a sustained release pharmaceutical composition at a dissolution rate of tacrolimus after 4 hours from the beginning of a dissolution test of less than 35%;
17. a method of regulating a ratio of a maximum blood tacrolimus concentration (Cmax) when administered after eating a meal to a maximum blood tacrolimus concentration when administered in a fasted state to 0.7 or more, and/or a ratio of an area under a blood tacrolimus concentration versus time curve (AUC) when administered after eating a meal to an area under a blood tacrolimus concentration versus time curve when administered in a fasted state to 0.8 or more, by dissolving tacrolimus contained in a sustained release pharmaceutical composition at a dissolution rate of tacrolimus after 4 hours from the beginning of a dissolution test of less than 35%;
18. a method of regulating a ratio of a maximum blood tacrolimus concentration (Cmax) to a blood tacrolimus concentration after approximately 8 hours from oral administration of tacrolimus (C8h) to 5 or less, by dissolving tacrolimus contained in a sustained release pharmaceutical composition at a dissolution rate of tacrolimus after 4 hours from the beginning of a dissolution test of less than 35%; and
19. a method of regulating a ratio of a maximum blood tacrolimus concentration (Cmax) to a blood tacrolimus concentration after approximately 24 hours from oral administration of tacrolimus (Cmin) to 3 or less, by dissolving tacrolimus contained in a sustained release pharmaceutical composition at a dissolution rate of tacrolimus after 4 hours from the beginning of a dissolution test of less than 35%.

### EFFECTS OF THE INVENTION

According to the present invention, a sustained release pharmaceutical composition for tacrolimus capable of inhibiting the change of PK parameters by the intake of food and obtaining a profile showing a low PT ratio and constant blood concentrations can be provided.

### BEST MODE FOR CARRYING OUT THE INVENTION

The sustained release pharmaceutical composition for tacrolimus of the present invention contains, at least, a solid dispersion of tacrolimus, and one or more sustained release bases (such as a water-soluble polymer, a gum base, a membrane forming agent, or the like) which do not form the solid dispersion. In the sustained release pharmaceutical composition for tacrolimus of the present invention, a dissolution rate after 4 hours from the beginning of a dissolution test may be less than 35%, preferably 10% or more and less than 35%. Further, a dissolution rate after 24 hours may be 60% or more, preferably 70% or more.

Tacrolimus [chemical name: 17-allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone], which is the active ingredient of the solid dispersion of tacrolimus used in the present invention, may be obtained by isolation and purification from a culture of Streptomyces tsukubaensis, for example, in accordance with a method disclosed in Japanese Patent Publication (Kokai) No. 62-277321.

The solid dispersion as used herein means a composition in which a drug in an amorphous form is dispersed in a crystalline carrier. As the solid dispersion of tacrolimus, for example, a tacrolimus-containing composition disclosed in Japanese Patent Publication (Kokai) No. 62-277321 may be exemplified. This tacrolimus-containing composition contains tacrolimus and a water-soluble polymer as a base (hereinafter referred to as a solid base), and, if desired, may further contain various additives which are conventionally used in the field of pharmaceutical preparations, such as a filler, a disintegrator, a coloring agent, a sweetener, a flavor, a diluent, or a lubricant.

The solid dispersion of tacrolimus used in the present invention may be prepared in accordance with, for example, a method disclosed in Japanese Patent Publication (Kokai) No. 62-277321. More particularly, the solid dispersion of tacrolimus may be prepared by dissolving tacrolimus in an organic solvent (for example, a lower alcohol, such as methanol, ethanol, propanol, isopropanol, or the like, ethyl acetate, or diethyl ether), adding a water-soluble polymer, adding one or more additives to the resulting suspension or solution if desired, and then removing the organic solvent from the mixture.

The water-soluble polymer is not particularly limited, so long as tacrolimus or a pharmaceutically acceptable salt thereof may be dispersed. Examples of the water-soluble polymer include, for example, a water-soluble cellulose polymer, such as hydroxypropylmethyl cellulose, hydroxypropyl cellulose, methyl cellulose, or the like. The content of the water-soluble polymer is not particularly limited, so long as tacrolimus or a pharmaceutically acceptable salt thereof may be dispersed, and is preferably 0.1:1 to 20:1 as a ratio by weight of the water-soluble polymer to tacrolimus (water-soluble polymer:tacrolimus).

As other preferred solid bases which may be used in the present invention, a water-insoluble base may be exemplified. For example, a solid dispersion in which tacrolimus is present in an amorphous state in a solid base composed of ethyl cellulose and hydroxypropylmethyl cellulose may be prepared in accordance with a method disclosed in WO 99/49863. Further, aminoalkylmethacrylate copolymer E such as Eudragit E (product name; degussa) may be used as a solid base. Furthermore, examples of other solid dispersions include, for example, a solid dispersion which contains various polymeric bases and is obtained by a twin screw extruder (WO 2003/077827), a solid dispersion with a water-insoluble polymer such as a biodegradable polymer (WO 2003/043603, WO 2004/000279, or WO 2004/071494), a solid dispersion with a lipid (WO 2003/013566, WO 2004/009075, WO 2004/087052, WO 98/40051, or WO 99/00113), a solid dispersion with a self-emulsifying agent (WO 2005/030169), a solid dispersion with an extract (WO 2003/049753, WO 2004/009061, or WO 2004/067018), a solid dispersion obtained by solubilization with phospholipid micelle (WO 99/44642), a solid dispersion obtained by spray drying with an O/W emulsion (WO 2004/062643), or the like.

A carrier for a sustained release pharmaceutical composition, which is contained in the pharmaceutical composition of the present invention and orally administered together with tacrolimus or a pharmaceutically acceptable salt thereof, is not particularly limited, so long as it is a carrier, a pharmaceutical formulation, or a technique for manufacturing pharmaceutical preparations capable of maintaining tacrolimus in a concentration effective in treating or preventing diseases.
Examples of such a carrier (or a pharmaceutical formulation, or a technique for manufacturing pharmaceutical preparations) which forms the composition or components in the present invention include, for example, (A) a sustained release hydrogel-forming formulation in which the formulation is almost completely gelled during the retention in the stomach and the small intestine of the upper digestive tract and the drug can be released in the colon of the lower digestive tract, (B) an osmotic pump type formulation, (C) a gel formulation in which a plurality of gums is combined, (D) a multi-layered tablet formulation consisting of a drug core and a release-controlling layer which are geometrically arranged, (E) a formulation utilizing a swelling polymer, (F) a matrix formulation utilizing a water-soluble polymer, (G) a sustained release formulation with a coating membrane, and the like, as described in detail below. The compositions relating to these techniques for manufacturing pharmaceutical preparations, and the techniques per se are incorporated herein by reference.
The sustained release pharmaceutical composition for tacrolimus of the present invention includes, for example,
(1) a sustained release hydrogel-forming formulation,
(2) an osmotic pump type formulation,
(3) a formulation utilizing a swelling polymer,
(4) a matrix formulation utilizing a water-soluble polymer,
(5) a sustained release formulation with a coating membrane,
(6) a multi-layered formulation consisting of a drug core and a release-controlling layer which are geometrically arranged, and
(7) a gel formulation in which a plurality of gums is combined. These embodiments can exhibit sufficient effects of the present invention, and the sustained release hydrogel-forming formulation is most preferable.

A drug-releasing property of the sustained release pharmaceutical composition for tacrolimus of the present invention can be evaluated by known dissolution tests, particularly a dissolution test, method 2 (paddle method), described in the Japanese Pharmacopoeia. When this method is selected, 900 mL of a solution prepared by dissolving 0.005% of hydroxypropyl cellulose (HPC) in a phosphate buffer (pH4.5) or a second fluid (JP2) of a disintegration test described in the Japanese Pharmacopoeia is used as a test medium, and the test is carried out at a paddle rotation speed of 100 rpm without the use of a sinker. Samples are collected at predetermined times, and amounts of tacrolimus in the sampling solutions are measured using an HPLC with an ultraviolet and visible detector (a detecting wavelength = 210 nm).

Other dissolution tests may be used, so long as the above test medium or a similar test medium is used. For example, a USP Dissolution, Rotating paddle method (Apparatus 2) or the like may be exemplified.

An administration in a fasted state as used herein means that a drug is administered to a subject which has been fasted for at least 8 hours. An administration after eating a meal means that a drug is administered within approximately 30 minutes after the intake of food.

To avoid food effects as used herein means that the change of pharmacokinetics by food intake is decreased, when a tacrolimus formulation is orally administered. The change of pharmacokinetics by food intake can be evaluated by, for example, a maximum blood drug concentration (Cmax) and/or an area under a blood drug concentration versus time curve (AUC). For example, the evaluation can be carried out by calculating maximum blood drug concentrations and/or areas under a blood drug concentration versus time curve in a fasted state and after eating a meal, and comparing the calculated values with each other.

More particularly, the evaluation can be carried out by determining a maximum blood drug concentration (a) when a drug is administered in a fasted state, and a maximum blood drug concentration (b) when the drug is administered following a meal, and calculating the ratio (b/a). An appropriate ratio varies dependently on the type of a drug or the kind of an animal, but it can be judged that the effects by food intake is small when the ratio is close to 1. Similarly, the effects by food intake can be evaluated by, for example, an area under a blood drug concentration versus time curve (AUC). For example, the effects by food intake can be evaluated by calculating each AUC in a fasted state and after eating a meal, and comparing the calculated values with each other. More particularly, the effects by food intake can be evaluated by determining an AUC (c) when a drug is administered in a fasted state, and an AUC (d) when the drug is administered following a meal, and calculating the ratio (d/c). In the same way as a maximum blood drug concentration, it can be judged that the effects by food intake is small when the ratio is close to 1.

In the sustained release pharmaceutical composition for tacrolimus of the present invention, a ratio (b/a) of a maximum blood drug concentration (b) when administered after eating a meal to a maximum blood drug concentration (a) when administered in a fasted state is 0.3 or more and 2.0 or less, preferably 0.7 or more and 1.4 or less.

In the sustained release pharmaceutical composition for tacrolimus of the present invention, a ratio (d/c) of an area under a blood drug concentration versus time curve (AUC) (d) when administered after eating a meal to an area under a blood drug concentration versus time curve (c) when administered in a fasted state is 0.5 or more and 2.0 or less, preferably 0.8 or more and 1.3 or less.

To improve safety profile as used herein means that a range of the variation in blood concentrations is decreased, when a tacrolimus formulation is orally administered. The range of the variation in blood concentrations can be evaluated, for example, by comparing a maximum blood drug concentration (Cmax) with a blood drug concentration (Ct) at a given time (t) after the administration.

More particularly, the evaluation can be carried out by determining a maximum blood drug concentration (e) and a blood drug concentration (f) at the final point in time when a blood drug concentration can be detected after the administration, and calculating the ratio (e/f). An appropriate ratio varies dependently on the type of a drug or the kind of an animal, but it can be judged that the range of the change in blood concentrations is small when the ratio is close to 1.
In this connection, the final point in time when a blood drug concentration can be detected varies dependently various conditions, but is preferably 8 hours or 24 hours.

In the sustained release pharmaceutical composition for tacrolimus of the present invention, a ratio (e/f) of a maximum blood drug concentration (e) to a blood drug concentration (f) at the final point in time when a blood drug concentration can be detected after the administration is 5 or less, preferably 3 or less.

Hereinafter, each embodiment of the sustained release pharmaceutical composition for tacrolimus of the present invention will be explained in detail.

### (1) Sustained release hydrogel-forming formulation

The sustained release hydrogel-forming formulation contains, as the carrier for a sustained release pharmaceutical composition, an additive that allows water to penetrate into the formulation (designated as a gelling agent, a promoting agent for gelling, or a hydrophilic base, but hereinafter referred to as hydrophilic base), and a polymer which forms a hydrogel (hereinafter referred to as hydrogel-forming polymer).

The hydrophilic base is not particularly limited, so long as it can be dissolved before the gelling of a polymer which forms a hydrogel used in the pharmaceutical composition. In the hydrophilic base, the amount of water necessary to dissolve 1 g of the base is preferably 5 mL or less (20±5°C), more preferably 4 mL or less (the same temperature). When the base has a high solubility to water, the effect that allows water to penetrate into the formulation is high. Examples of the hydrophilic base are, for example, water-soluble polymers, such as polyethylene glycol [PEG: for example, PEG 400, PEG 1500, PEG 4000, PEG 6000, PEG 20000 (product name, manufactured by Sanyo Chemical Industries, Ltd.)], and polyvinyl pyrrolidone (PVP: for example, PVP K30 (product name, manufactured by BASF); sugar alcohols, such as D-sorbitol, xylitol, and the like; saccharides, such as sucrose, anhydrous maltose, D-fructose, dextran (for example, Dextran 40), glucose, and the like; surfactants, such as polyoxyethylene hydrogenated castor oil [HCO: for example, Cremophor RH40 (manufactured by BASF), HCO-40, HCO-60 (manufactured by Nikko Chemicals)], polyoxyethylene polyoxypropylene glycol [for example, Pluronic F68 (manufactured by Asahi Denka) and the like], polyoxyethylene sorbitan higher fatty acid esters [Tween: for example, Tween 80 (manufactured by Kanto Chemical)], and the like; salts, such as sodium chloride, magnesium chloride, and the like; organic acids, such as citric acid, tartaric acid, and the like; amino acids, such as glycine, β-alanine, lysine hydrochloride, and the like; and aminosaccharides, such as meglumine and the like. Preferred hydrophilic bases include PEG 6000, PVP, D-sorbitol, and the like. These hydrophilic bases may be used alone, or as a combination of two or more members.

The content of the hydrophilic base may be appropriate selected in accordance with various factors, such as properties (solubility, therapeutic effect, and the like) and content of a drug, solubility of the hydrophilic base, properties of a hydrogel-forming polymer, conditions of a subject to be administered, and the like, but an amount in which gelling is almost completely achieved during the retention of the formulation in the upper digestive tract is preferred. A retention time of a drug in the upper digestive tract varies according to species and individuals, but those after administration in dogs and humans are approximately 2 hours and approximately 4 to 5 hours, respectively [Br.J.Clin.Pharmac., (1988) 26, 435-443]. In humans, the content of the hydrophilic base is preferably an amount in which gelling of the formulation is almost completely achieved after 4 to 5 hours from the administration thereof. The content is generally approximately 5 to 80 W/W%, preferably approximately 5 to 60 W/W%, with respect to the weight of the formulation. When the content of the hydrophilic base is low, the inside of the formulation is not gelled, and sufficient release is not achieved in the colon. By contrast, when the content is high, gelling is completed in a short time, but the gel is easily disintegrated to cause an increased releasing rate of drug and insufficient sustained release. In addition, the size of the formulation is increased, because of an increasing content of the base.

The hydrogel-forming polymer used has properties, such as viscosity and the like at the time of gelling, that maintain the form of the almost completely gelled formulation against the motility of the digestive tract accompanied by food digestion, and migrate the gelled formulation to the colon in the lower digestive tract while maintaining the shape to a certain extent.

Preferred hydrogel-forming polymers have a high viscosity at the time of gelling. For example, polymers having a viscosity of 1000 cps or more in a 1% aqueous solution (25°C) is preferred. Further, since properties of polymers depend on the molecular weight (weight average molecular weight) thereof, preferred hydrogel-forming polymers applicable to the formulation are those having a higher molecular weight, preferably an average molecular weight of 2,000,000 or more, more preferably an average molecular weight of 4,000,000 or more. Examples of such polymers are, for example, polyethylene oxide (PEO) having a molecular weight of 2,000,000 or more [such as Polyox (product name) WSR-303 (average molecular weight: 7,000,000, viscosity: 7500 to 10000 cps in a 1% aqueous solution at 25°C), Polyox WSR Coagulant (average molecular weight: 5,000,000, viscosity: 5500 to 7500 cps in the same), Polyox WSR-301 (average molecular weight of 4,000,000, viscosity: 1650-5500 cps in the same), Polyox WSR-N-60K (average molecular weight: 2,000,000, viscosity: 2000 to 4000 cps in a 2% aqueous solution at 25°C) (all manufactured by Dow Chemical)]; hydroxypropylmethyl cellulose (HPMC) [such as Metolose (product name) 90SH100000 (viscosity: 4100 to 5600 cps in a 1% aqueous solution at 20°C), Metolose 90SH50000 (viscosity: 2900 to 3900 cps in the same), Metolose 90SH30000 (viscosity: 25000 to 35000cps in a 2% aqueous solution at 20°C) (all manufactured by Shin-Etsu Chemical Co., Ltd.)]; carboxymethylcellulose sodium (CMC-Na) [such as Sunrose (product name) F-150MC (average molecular weight: 200,000, viscosity: 1200 to 1800 cps in a 1% aqueous solution at 25°C), Sunrose F-1000MC (average molecular weight: 420,000, viscosity: 8000 to 12000cps in the same), Sunrose F-300MC (average molecular weight: 300,000, viscosity: 2500 to 3000cps in the same) (all manufactured by Nippon Paper Chemicals Co., Ltd.)]; hydroxyethyl cellulose (HEC) [such as HEC Daicel (product name) SE850 (average molecular weight: 1,480,000, viscosity: 2400 to 3000 cps in a 1% aqueous solution at 25°C), HEC Daicel SE900 (average molecular weight: 1,560,000, viscosity: 4000 to 5000 cps in the same) (all manufactured by Daicel chemical Industries, Ltd.)]; carboxyvinyl polymers [such as Carbopole 940 (average molecular weight: approximately 2,500,000) (manufactured by B.F.Goodrich Chemical); and the like. PEO having an average molecular weight of 2,000,000 or more is preferred. When the release is maintained for a long time, such as for 12 or more hours, suitable polymers include those having a higher molecular weight, preferably an average molecular weight of 4,000,000 or more, and those having a higher viscosity, preferably a viscosity of 3000 cps in a 1% aqueous solution at 25°C. Such hydrogel-forming polymers may be used alone, or as a mixture of two or more of the members. A mixture consisting of two or more polymers and having the above-mentioned properties as a whole may be suitably used as the hydrogel-forming polymer.

To release a drug in the colon of a human, a portion of the gelled formulation should be remained in the colon after at least 6 to 8 hours, preferably 12 hours or more, from the administration of the formulation. The preparation of hydrogel-forming formulations having such properties varies according to the size of the formulation, the kind of polymers, properties of a drug and an additive that allows water to penetrate into the formulation, the contents thereof, and the like. For a formulation of 600 mg or less per tablet, the content of the hydrogel-forming polymer with respect to the weight of the formulation is generally 10 to 95 W/W%, preferably 15 to 90 W/W%, and the content per tablet is generally 40 mg or more, preferably 70 mg or more, more preferably 100 mg or more. When the content is lower than these values, there is a possibility that sufficient sustained release is not achieved, due to erosion in the digestive tract for a long time.

When polyethylene oxide is used as the hydrogel-forming polymer, it is preferable to add yellow ferric oxide and/or red ferric oxide in an amount that does not sequentially change the releasing properties of the drug.
Yellow ferric oxide or red ferric oxide may be used alone or as a mixture.
The addition may be carried out by physical mixing, film coating to a drug core or the like, and the like.

The content of yellow ferric oxide and/or red ferric oxide is not particularly limited, so long as it is an amount that does not change the releasing properties of tacrolimus used in the present invention. The content is preferably 1 to 20 W/W%, more preferably 3 to 15 W/W%, to the weight of the formulation. When yellow ferric oxide and/or red ferric oxide are added by physical mixing, the content is preferably 1 to 20 W/W%, more preferably 3 to 15 W/W%, to the weight of the formulation. For red ferric oxide, the content is preferably 5 to 20 W/W%, more preferably 10 to 15 W/W%, to the weight of the formulation. For yellow ferric oxide, the content is preferably 1 to 20 W/W%, more preferably 3 to 10 W/W%. When coated by film coating, the content is preferably 0.3 to 2 W/W%, more preferably 0.5 to 1.5 W/W%, to the weight of the formulation. In this case, the concentration of yellow ferric oxide or red ferric oxide contained in the film is preferably 5 to 50 W/W%, more preferably 10 to 20 W/W%.

The "physical mixing" as used herein means a method in which components used are uniformly mixed, for example, a method in which a drug, polyethylene oxide, and the ferric oxide(s) are uniformly mixed, and as a result, the drug and the ferric oxide are uniformly dispersed in PEO as the main base of the sustained release formulation. The term "film coating" means that a formulation as the core of a tablet or the like is coated, for example, that the ferric oxide(s) is dissolved or suspended in a water-soluble polymer solution of hydroxypropylmethyl cellulose or the like, and tablets that have been separately prepared are coated with this solution or suspension to form a thin layer. Yellow ferric oxide and/or red ferric oxide can exist at any place in the formulation. It can be contained, for example, in the film by film coating or the like, in granules by granulation or the like, in the matrix (for example, around polyethylene oxide), or the like.

The preparation of the sustained release hydrogel-forming formulation, an embodiment of the sustained release pharmaceutical composition of the present invention, is not particularly limited. The formulation may be prepared by, for example, a tableting method in which the drug, the hydrophilic base, and the hydrogel-forming polymer, and additives such as yellow ferric oxide and/or red ferric oxide if desired, are mixed, and the mixture is compression molded; capsule compress on filling; extrusion molded, or injection molded, in which the mixture is melted and solidified; or the like. In addition, coating, such as conventional sugar coating, film coating, and the like after molding, can be optionally performed. Capsules may be filled with the molded product.

### (2) Osmotic pump type formulation

Osmotic pump type formulations utilize osmotic pressure to generate a driving force for imbibing fluid into a formulation, by a semipermeable membrane that permits free diffusion of fluid but not a drug or an osmoagent. The osmotic systems are **characterized in that** the action thereof is pH-independent, and a drug can be sustainedly released at a constant rate for a long time, even as the formulation transits the gastrointestinal tract and encounters environments having different pH values.

Such osmotic pump type formulations are reported in Santus and Baker, "Osmotic drug delivery: a review of the patent literature", Journal of Controlled Release, 35, p.1-21, (1995). Further, osmotic pump type formulations are described in U.S. Patents Nos. 3,845,770, 3,916,899, 3,995,631, 4,008,719, 4,111,202, 4,160,020, 4,327,725, 4,519,801, 4,578,075, 4,681,583, 5,019,397, and 5,156,850, the contents of which are incorporated herein by reference.

In the osmotic pump type formulation of the present invention, a bilayered compressed core consisting of a drug layer containing tacrolimus or a pharmaceutical acceptable salt thereof, and a push layer, is coated with a semipermeable membrane that permits water or outer fluid but not a drug, an osmoagent, an osmopolymer, or the like. The semipermeable membrane is provided with at least one drug delivery orifice for connecting the inside of the formulation with the exterior environment. Therefore, after the osmotic pump type formulation is orally administered, fluid such as water transits the semipermeable membrane, and penetrates into the inside of the formulation. As a result, an osmotic action is generated, and tacrolimus is sustainedly released through the drug delivery orifice(s) at a constant rate for a long time.

The drug layer contains tacrolimus or a pharmaceutically acceptable salt thereof, as a mixture with a pharmaceutically acceptable additive(s).

The push layer contains one or more osmotic active components, but does not contain tacrolimus or a pharmaceutically acceptable salt thereof, as described in detail below. Typical osmotic active component(s) contained in the push layer may be composed of an osmoagent and one or more osmopolymers. The osmopolymer as used herein means a polymer which has relatively a large molecular weight and swells when fluid is imbibed, to release tacrolimus through the drug delivery orifice(s).

The semipermeable membrane used is not particularly limited, so long as it is permeable to the passage of an external fluid, such as water and biological fluids, and substantially impermeable to the passage of tacrolimus, an osmoagent, an osmopolymer, and the like. Such a semipermeable membrane is essentially nonerodible, and insoluble in a living body.

As polymers for forming the semipermeable membrane, for example, semipermeable homopolymers, semipermeable copolymers, and the like may be used. As materials for such polymers, cellulosic polymers, such as cellulose esters, cellulose ethers, cellulose ester-ethers, and the like, may be used. The cellulosic polymers have a degree of substitution (DS) of anhydroglucose units of more than 0 and 3 or less. The degree of substitution (DS) means the average number of hydroxyl groups originally present on the anhydroglucose units that are replaced by a substituting group or converted into another group. The anhydroglucose unit can be partially or completely substituted with groups, such as acyl, alkanoyl, alkenoyl, aroyl, alkyl, alkoxy, halogen, carboalkyl, alkylcarbamate, alkylcarbonate, alkylsulfonate, alkylsulfamate, semipermeable polymer forming groups, and the like, wherein the organic moieties contain 1 to 12 carbon atoms, preferably 1 to 8 carbon atoms.

As the typical semipermeable compositions, one member, or two or more members selected from the group consisting of cellulose acylate, cellulose diacylate, cellulose triacylate, cellulose acetate, cellulose diacetate, cellulose triacetate, mono-, di-, and tri-cellulose alkanylates, mono-, di-, and tri-alkenylates, mono-, di-, and tri-aroylates, and the like, may be used. Representative polymers include cellulose acetate having a DS of 1.8 to 2.3 and an acetyl content of 32 to 39.9%; cellulose diacetate having a DS of 1 to 2 and an acetyl content of 21 to 35%; cellulose triacetate having a DS of 2 to 3 and an acetyl content of 34 to 44.8%; and the like.

More specific cellulosic polymers include cellulose propionate having a DS of 1.8 and a propionyl content of 38.5%; cellulose acetate propionate having an acetyl content of 1.5 to 7% and an acetyl content of 39 to 42%; cellulose acetate propionate having an acetyl content of 2.5 to 3%, an average propionyl content of 39.2 to 45%, and a hydroxyl content of 2.8 to 5.4%; cellulose acetate butyrate having a DS of 1.8, an acetyl content of 13 to 15%, and a butyryl content of 34 to 39%; cellulose acetate butyrate having an acetyl content of 2 to 29%, a butyryl content of 17 to 53%, and a hydroxyl content of 0.5 to 47%; cellulose triacylates having a DS of 2.6 to 3, such as cellulose trivalerate, cellulose trilamate, cellulose tripalmitate, cellulose trioctanoate, and cellulose tripropionate; cellulose diesters having a DS of 2.2 to 2.6, such as cellulose disuccinate, cellulosedipalmitate, cellulose dioctanoate, cellulose dicaprylate, and the like; mixed cellulose esters, such as cellulose acetate valerate, cellulose acetate succinate, cellulose propionate succinate, cellulose acetate octanoate, cellulose valerate palmitate, cellulose acetate heptanoate, and the like. Semipermeable polymers are disclosed in U.S. Patent No. 4,077, 407, and can be synthesized and obtained by procedures described in Encyclopedia of Polymer Science and Technology, Vol. 3, pp. 325-354 (1964), Interscience PublishersInc., New York, NY. The content of the polymers is not particularly limited, so long as it is an amount permeable to the passage of an external fluid, such as water and biological fluids, and substantially impermeable to the passage of tacrolimus, an osmoagent, an osmopolymer, and the like. The content of the polymers is preferably 6 to 20 W/W%, more preferably 8 to 18 W/W%, with respect to the weight of a dilayered compressed core consisting of a drug layer and a push layer.

Semipermeable polymers for forming the semipermeable membrane further include cellulose acetaldehyde dimethyl acetate; cellulose acetate ethylcarbamate; cellulose acetate methyl carbamate; cellulose dimethylaminoacetate; semipermeable polyurethanes; semipermeable sulfonate polystyrenes; cross-linked selectively semipermeable polymers formed by the coprecipitation of an anion and a cation, as disclosed in U.S. Patents Nos. 3,173,876, 3,276,586, 3,541,005, 3,541,006, and 3,546,142; semipermeable polymers, as disclosed in U.S. Patent No. 3,133,132; semipermeable polystyrene derivatives; semipermeable poly (sodium styrenesulfonate); semipermeable poly (vinylbenzyltrimethylammonium chloride); and semipermeable polymers exhibiting a fluid permeability of 10⁻⁵ to 10⁻² (cc mL/cm hr atm), expressed as hydrostatic or osmotic pressure differences per atmosphere across a semipermeable membrane. These polymers are described in U.S. Patents Nos. 3,845,770, 3,916,899, and 4,160,020, and in Handbook of Common Polymers, Scott and Roff (1971) CRC Press, Cleveland, OH.

The semipermeable membrane may contain a flux-regulating agent. The flux-regulating agent means a substance added to assist in regulating the fluid permeability or flux through the semipermeable membrane. The flux-regulating agents include a substance which enhances the flux (hereinafter referred to as flux-enhancing agent) and a substance which decreases the flux (hereinafter referred to as flux-decreasing agent). The flux-enhancing agents are essentially hydrophilic, while the flux-decreasing agents are essentially hydrophobic. The flux-regulating agents include, for example, polyhydric alcohols, polyalkylene glycols, polyalkylenediols, polyesters of alkylen glycols, and the like.

Typical flux-enhancing agents include polyethylene glycols 300, 400, 600, 1500, 4000, 6000 and the like; low molecular weight glycols, such as polypropylene glycol, polybutylene glycol, and polyamylene glycol: polyalkylenediols, such as poly(1,3-propanediol), poly(1, 4-butanediol), poly(1,6-hexanediol), and the like; fatty acids, such as 1,3-butylen glycol, 1,4-pentamethylene glycol, 1,4-hexamethylene glycol, and the like; alkylen triols, such as glycerine, 1,2,3-butanetriol, 1,2,4-hexanetriol, 1,3,6-hexanetriol, and the like; esters, such as ethylene glycol dipropionate, ethylene glycol butyrate, butylene glycol dipropionate, glycerol acetate esters, and the like. Preferred flux-enhancing agents include difunctional block-copolymers of propylene glycol, polyoxyalkylene or derivatives thereof, known as pluronics (trademark, BASF).
Typical flux-decreasing agents include phthalates substituted with an alkyl or alkoxy or with both an alkyl and alkoxy group such as diethyl phthalate, dimethoxyethyl phthalate, dimethyl phthalate, and [di(2-ethylhexyl) phthalate], and aryl phthalates such as triphenyl phthalate and butyl benzyl phthalate; insoluble salts such as calcium sulfate, barium sulfate, calcium phosphate, and the like; insoluble oxides such as titanium oxide; polymers in the form of powder, granules, and the like, such as polystyrene, polymethylmethacrylate, polycarbonate, and polysulfone; esters such as citric acid esters esterified with long chain alkyl groups; inert and water impermeable fillers; resins compatible with cellulose based semipermeable membrane forming materials; and the like.

The content of the flux-regulating agent contained in the semipermeable membrane is approximately 0.01 to approximately 20 W/W% or more.

Other substances may be contained in the semipermeable membrane to impart plasticity, flexibility, and elongation properties, to make the membrane less brittle, and to render tear strength. Such substances include phthalate plasticizers such as dibenzyl phthalate, dihexyl phthalate, butyl octyl phthalate, phthalates having 6 to 11 straight chain carbon atoms, di-isononyl phthalte, di-isodecyl phthalate, and the like. Other plasticizers include nonphthalates such as triacetin, dioctylazelate, epoxidized tallate, tri-isoctyl trimellitate, tri-isononyl trimellitate, sucrose acetate isobutyrate, epoxidized soybean oil, and the like.

The content of the plasticizer contained in the semipermeable membrane is approximately 0.01 to 20 W/W% or more.

The push layer is in contacting layered arrangement with the drug layer. The push layer contains an osmopolymer that imbibes an aqueous or biological fluid and swells to push tacrolimus or a pharmaceutically acceptable salt thereof through the exit means of the formulation. The osmopolymer as used herein means a polymer that interacts with water or aqueous biological fluids and swells or expands to a high degree. Preferred osmopolymers are swellable and hydrophilic polymers exhibiting a 2 to 50-fold volume increase. The osmopolymer can be non-crosslinked or crosslinked, but is preferably at least lightly crosslinked in a preferred embodiment, to create an extended polymer network that is too large to exit the formulation. The content of the osmopolymer can be appropriately selected in accordance with various factors such as properties, content, and the like of a drug contained in the drug layer, but is not particularly limited, so long as it is an amount capable of releasing the drug from the drug layer at a desired dissolution rate by swelling. The amount is preferably 30 mg or more, more preferably 50 mg or more. The content is 40 to 80 W/W% with respect to the weight of the push layer.

The osmopolymers include one or more members selected from the group consisting of poly(alkylen oxide) having a number average molecular weight of 1,000,000 to 15,000,000, as represented by polyethylene oxide, and poly(alkali carboxymethylcellulose) having a number average molecular weight of 500,000 to 3,500,000, wherein the alkali is sodium, potassium, or lithium. The osmopolymers further include osmopolymers comprising polymers that form hydrogels, such as Carbopole (registered trademark), acidic carboxypolymers, polymers of acrylic cross-linked with polyallyl sucrose (known as carboxypolymethylene), and carboxyvinyl polymers having a molecular weight of 250,000 to 4,000,000; Cyanamer (registered trademark) polyacrylamides; cross-linked water swellable indenemaleic anhydride polymers; Good-rite (registered trademark) polyacrylic acid having a molecular weight of 80,000 to 200,000; Aqua-Keeps (registered trademark), acrylate polymer polysaccharides composed of condensed glucose units, such as diester cross-linked polygluran; and the like. Polymers that form hydrogels are described in U.S. Patents Nos. 3,865,108, 4,002,173, and 4,207,893, and in Handbook of Common Polymers, Scott and Roff, Chemical Rubber Co., Cleveland, OH.

The osmoagent (sometimes referred to as an osmotic solute or an osmotically effective agent) may be contained in both of the drug layer containing tacrolimus or a pharmaceutically acceptable salt thereof and the push layer, and is not particularly limited, so long as it exhibits an osmotic activity gradient across the semipermeable membrane. Suitable osmagents include a member or two or more members selected from the group consisting of sodium chloride, potassium chloride, lithium chloride, magnesium sulfate, magnesium chloride, potassium sulfate, sodium sulfate, lithium sulfate, potassium acid phosphate, mannitol, glucose, lactose, sorbitol, inorganic salts, organic salts, and carbohydrates. The content of the osmoagent used is 15 to 40 W/W% with respect to the weight of the push layer.

Solvents suitable for manufacturing the formulation components include aqueous or inert organic solvents that do not adversely harm the substances used in the system. Such solvents broadly include one or more members selected from the group consisting of aqueous solvents, alcohols, ketones, esters, ethers, aliphatic hydrocarbons, halogenated solvents, cycloaliphatic solvents, aromatic solvents, heterocyclic solvents, and mixtures thereof. Typical solvents include acetone, diacetone alcohol, methanol, ethanol, isopropyl alcohol, butyl alcohol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloride, nitroethane, nitropropane, tetrachloroethane, ethyl ether, isopropyl ether, cyclohexane, cyclooctane, benzene, toluene, naphtha, 1,4-dioxane, tetrahydrofuran, diglyme, water, aqueous solvents containing inorganic salts (such as sodium chloride, calcium chloride, and the like), and mixtures thereof (such as acetone and water, acetone and methanol, acetone and ethyl alcohol, methylene dichloride and methanol, and ethylene dichloride and methanol).

The drug layer is formed from a pharmaceutical composition consisting of tacrolimus or a pharmaceutically acceptable salt thereof in an amount pharmacologically effective in treatment or prevention, and a carrier for a sustained release pharmaceutical composition. The carrier for a sustained release pharmaceutical composition may include hydrophilic polymers.
The hydrophilic polymers impart an action of releasing tacrolimus at a constant releasing rate. Suitable hydrophilic polymers include poly(alkylene oxide) having a number average molecular weight of 100,000 to 750,000, such as poly(ethylene oxide), poly(methylene oxide), poly(buthylene oxide, and poly(hexylene oxide); and poly(carboxymethyl cellulose) having a number average molecular weight of 40,000 to 400,000, typically poly(alkali carboxymethyl cellulose), poly(sodium carboxymethyl cellulose), poly(potassium carboxymethyl cellulose), and poly(lithium carboxymethyl cellulose). The drug composition may contain hydroxypropylalkyl cellulose having a number average molecular weight of 9,200 to 125,000, typically hydroxypropylethyl cellulose, hydroxypropylmethylcellulose, hydroxypropylbutyl cellulose, and hydroxypropylpentyl cellulose, to improve delivery properties of the formulation; and polyvinylpyrrolidone having a number average molecular weight of 7,000 to 75,000, to improve flow properties of the formulation. Among these polymers, poly(ethylene oxide) having a number average molecular weight of 100,000 to 300,000 is most preferable. The content of the hydrophilic polymer can be appropriately selected in accordance with various factors such as physicochemical properties, content, and the like of a drug contained, but is 40 to 90 W/W% with respect to the drug layer.

The drug layer may further contain surfactants and disintegrants, if desired. Suitable surfactants are those having an HLB value of approximately 10 to 25, such as polyethylene glycol 400 monostearate, polyoxyethylene-4-sorbitan monolaurate, polyoxyethylene-20-sorbitan monooleate, polyoxyethylene-20-sorbitan monopalmitate, polyoxyethylene-20-monolaurate, polyoxyethylene-40-stearate, sodium oleate, and the like. Disintegrants may be selected from starches, clays, celluloses, algins and gums and crosslinked starches, celluloses and polymers. Representative disintegrants include corn starch, potato starch, croscarmelose, crospovidone, sodium starch glycolate, Veegum HV, methylcellulose, agar, bentonite, carboxymethylcellulose, alginic acid, guar gum, and the like.

Pan coating may be used to prepare the completed formulation, except for the exit orifice for releasing a drug from the surface of the formulation. In the pan coating system, the composition for forming the semipermeable membrane is deposited by spraying the composition onto the surface of the bilayered compressed core formed from the drug layer and the push layer, accompanied by tumbling in a rotating pan. Alternatively, the compressed core may be coated with the semipermeable membrane by well-known techniques in the art. After the coating, the semipermeable membrane may be dried in a forced-air oven or in a temperature and humidity controlled oven to remove the solvent(s) used in the coating from the formulation. Drying conditions may be appropriately selected on the basis of an available equipment, ambient conditions, solvents, a coating agent, a coating thickness, and the like.

The osmotic pump type formulation, an embodiment of the sustained release pharmaceutical composition of the present invention, can be prepared by known conventional methods, such as wet granulation techniques. In the wet granulation, a drug and a carrier for a sustained release pharmaceutical composition are blended using an organic solvent, such as denatured absolute alcohol and the like, as a granulation solution. The remaining components may be dissolved in a portion of the granulation solution such as the above solvent, and a wet mixture separately prepared is gradually added to the drug mixture, accompanied by the continuous mixing in a blender. The granulation solution is added until a wet aggregate is generated, and the wet aggregate are sifted through a screen arranged on an oven tray. The mixture is dried at a temperature of approximately 24 to 35°C in a forced-air oven for approximately 18 to 24 hours. The dried granules are sized. A lubricant such as magnesium stearate or the like is added to the drug granules, and the whole is put into a milling jar and mixed on a jar mill for approximately 10 minutes. The composition is pressed into a layer, for example, in a Manestye (registered trademark) press or a Korsch LCT press. For a bilayered core, the drug-containing layer is pressed, and a composition for the push layer, prepared in a similar fashion by wet granulation techniques, is pressed against the drug-containing layer. One exit orifice, or two more exit orifices, are drilled in the drug layer end of the formulation. Optional water soluble overcoats, which may be colored (for example, Opadry colored coatings) or clear (for example, Opadry Clear), may be coated on the formulation to provide the completed formulation.

The osmotic pump type formulation, an embodiment of the sustained release pharmaceutical composition of the present invention, has at least one exit orifice. A drug is constantly released from the formulation through the exit orifice(s) by the compressed core. The exit orifice may be provided during the manufacture of the formulation, or during the drug delivery by the formulation in a fluid environment of use. The terms "exit orifice", "delivery exit", "drug delivery exit", and similar terms as used herein include terms selected from the group consisting of pass, opening, orifice, and bore. Further, these expressions include an orifice that is formed from a substance or polymer that erodes, dissolves or is leached from the outer wall.

This substance or polymer may include, for example, erodible poly(glycolic acid) or poly(lactic acid) in the semipermeable membrane; gelatinous filaments; water-removable poly(vinyl alcohol); a leachable compound, such as a fluid removable pore-forming substance selected from the group consisting of inorganic and organic salts, oxides, and carbohydrates. The exit(s) are formed by leaching one or two or more members selected from the group consisting of sorbitol, lactose, fructose, glucose, mannose, galactose, talose, sodium chloride, potassium chloride, sodium citrate and mannitol to provide a uniform-release dimensioned pore-exit orifice(s). The exit can have any shape, such as round, rectangle, square, elliptical, and the like, for the uniform release of a drug from the formulation. The formulation can be constructed with one or two or more exits in spaced-apart relation or on one or more surfaces of the formulation. The pore size of the exit is not particularly limited, so long as it can cooperate with the compressed core to control the release of the drug, but is preferably 0.3 to 0.6 mm. Drilling, including mechanical and laser drilling, through the semipermeable membrane can be used to form the exit orifice. Such exits and equipments for forming such exits are disclosed in U.S. Patent No. 3,916,899, by Theeuwes and Higuchi and in U.S. Patent No. 4,088,864, by Theeuwes, et al., each of which is incorporated herein by reference.

### (3) Formulation utilizing swelling polymer

The formulation utilizing a swelling polymer, as an embodiment of the sustained release pharmaceutical composition for tacrolimus of the present invention, is a sustained release formulation containing a water-soluble high molecular weight polymer which swells upon imbibition of water.

Formulation techniques using a swelling polymer which may be used in the sustained release pharmaceutical composition for tacrolimus of the present invention are described in U.S. Patents Nos. 6,340,475, 5,972,389, 5,582,837, and 5,007,790, the contents of which are incorporated herein by reference.

The "water-soluble high molecular weight polymer which swells upon imbibition of water" used is not particularly limited, so long as it is a pharmaceutically acceptable polymer that swells in a dimensionally unrestricted manner upon imbibition of water, and that releases a drug continuously. Suitable polymers are those having a weight average molecular weight of preferably approximately 4,500,000 or more, more preferably approximately 4,500,000 to approximately 10,000,000, most preferably approximately 5,000,000 to approximately 8,000,000.

Such polymers include cellulose polymers and derivatives thereof, polysaccharides and derivatives thereof, polyalkylene oxides, and crosslinked polyacrylic acids and derivatives thereof. The term "cellulose" as used herein means a linear polymer of anhydroglucose. Preferred cellulose polymers are alkyl-substituted cellulose polymers that dissolve in the gastrointestinal tract. Preferred alkyl-substituted cellulose derivatives are those substituted with alkyl groups having 1 to 3 carbon atoms each. Examples thereof include, for example, methylcellulose, hydroxymethylcellulose, hydroxyethylcellulose, hydroxypropylcellulose, hydroxypropylmethylcellulose, and carboxymethylcellulose. A preferred viscosity ranges between approximately 100 and approximately 110,000 cps, as measured in a 2% aqueous solution at 20°C. A viscosity in other embodiments ranges between approximately 1,000 and approximately 4,000 cps, as measured in a 1% aqueous solution at 20°C. More preferred alkyl-substituted celluloses are hydroxyethylcellulose and hydroxypropylmethylcellulose. Preferred hydroxyethylcellulose is NATRASOL (product name) 250H X NF.

Further, most preferred polymers are polyalkylene oxide derivatives, particularly polyethylene oxide, i.e., an unsubstituted linear polymer of ethylene oxide. Preferred polyethylene oxide has a weight average molecular weight of approximately 900,000 to approximately 8,000,000. A preferred viscosity ranges between approximately 50 to approximately 2,000,000 cps, as measured in a 2% aqueous solution at 20°C. Preferred polyethylene oxide is POLYOX (product name), such as grade WSR Coagulant and grade WSR 303.

Other examples of such polymers include both naturally-occurring and modified (semi-synthetic) polysaccharide gums, such as dextran, xanthan gum, gellan gum, welan gum, and rhamsan gum. Xanthan gum is preferred. Crosslinked polyacrylic acids of greatest utility are those whose properties are the same as those described above for alkyl-substituted celluloses and polyalkylene oxide polymers. Preferred crosslinked polyacrylic acids are those with a viscosity ranging from approximately 4,000 to approximately 40,000 cps, for a 1% aqueous solution at 25°C. Preferred examples are CARBOPOL (product name) NF grades 971P, 974P, and 934P, and WATER LOCK (product name) which are starch/acrylates/acrylamide copolymers.

The content of the "water-soluble high molecular weight polymer which swells upon imbibition of water" with respect to the weight of the formulation is not particularly limited, but is preferably approximately 1 to approximately 95 W/W%.

The formulation utilizing a swelling polymer, an embodiment of the sustained release pharmaceutical composition of the present invention, can be prepared as a pharmaceutically acceptable solid dosage form for oral administration such as tablets, particles, and particles retained in tablets or capsules. A presently preferred dosage form is a size 0 gelatin capsule containing two or three polymer particles (pellets) containing a drug. For the two-pellet capsules, the pellets are cylindrically shaped, 6.6 or 6.7 mm (or more generally, 6.5 to 7 mm) in diameter and 9.5 or 10.25 mm (or more generally, 9 to 12 mm) in length. For the three-pellet capsules, the pellets are cylindrically shaped, 6.6 mm in diameter and 7 mm in length. For a size 00 gelatin capsule with two pellets, the pellets are cylindrical, 7.5 mm in diameter and 11.5 mm in length. For a size 00 gelatin capsule with three pellets, the pellets are cylindrical, 7.5 mm in diameter and 7.5 mm in length. Another presently preferred dosage form is a tablet, with dimensions 18 to 22 mm in length, 6.5 to 7.8 mm in width, and 6.2 to 7.5 mm in height, more preferably with dimensions 20 mm in length, 6.7 mm in width, and 6.4 mm in height. These are merely examples, and the shapes and sizes can be varied considerably.

A particulate drug/polymer mixture or a drug-impregnated polymer matrix can be prepared by various known conventional methods, such as mixing, comminution, and fabrication techniques. These methods include, for example, direct compression using appropriate punches and dies, injection, and compression molding. When compression molding is carried out, lubricants may be optionally added. Examples of lubricants include stearic acid, magnesium stearate, calcium stearate, sodium stearyl fumarate, and the like, and magnesium stearate is preferred. The content of the lubricant is 0.25 to 3 W/W%, preferably less than 1 W/W%, with respect to the weight of the formulation. As other lubricants, hydrogenated vegetable oils, and hydrogenated and refined triglycerides of stearic and palmitic acids are preferable, and the content is approximately 1 to 5 W/W%, preferably approximately 2 W/W%, with respect to the weight of the formulation.

Most preferable sets of various components described above include a combination of approximately 90 to approximately 97 W/W% (with respect to the weight of the formulation) of polyethylene oxide having a weight average molecular weight of approximately 2,000,000 to approximately 7,000,000 as the "water-soluble high molecular weight polymer which swells upon imbibition of water" and less than approximately 2 W/W% (with respect to the weight of the formulation) of magnesium stearate as the lubricant. Examples of a combination of, for example, two water-soluble polymers include a combination of approximately 48 W/W% of polyethylene oxide having a weight average molecular weight of approximately 900,000 to approximately 7,000,000 and approximately 48 W/W% of hydroxypropylmethyl cellulose having a viscosity of approximately 3 to approximately 10,000 cps, as measured in a 2% aqueous solution at 20°C (weight ratio = about 1:1).
It is expected that the formulation utilizing a swelling polymer is retained in the stomach by swelling.

### (4) Matrix formulation utilizing water-soluble polymer

The matrix formulation utilizing water-soluble polymer, an embodiment of the sustained release pharmaceutical composition of tacrolimus of the present invention, is a sustained release formulation in which the drug is homogenously dispersed in one or more water-soluble polymers, such as hydroxypropylmethyl cellulose (HPMC).

Techniques for obtaining such a matrix formulation which may be used in the sustained release pharmaceutical composition of tacrolimus according to the present invention are disclosed, for example, in WO 93/16686, the contents of which are incorporated herein by reference.

When hydroxypropylmethyl cellulose, a water-soluble polymer, is brought into contact with water, hydration thereof is caused, and a hydrogel layer is formed on the surface of a matrix. This gel layer containing a drug formed on the matrix surface is gradually dissolved and eroded, to release the drug from the layer. The matrix formulation of the present invention is **characterized in that** a drug may be controllably released by repeating the contact with water, the formation of the gel layer containing the drug, and the dissolution and erosion of the gel layer.

The matrix formulation of the present invention is **characterized in that** a sustained release filler consisting of a water-soluble polymer, an inactive diluent, and a physiologically active substance are homogenously dispersed. The water-soluble polymer is not particularly limited, so long as it is gradually gelled, eroded, dissolved, and/or disintegrated when exposed to an environmental fluid. Examples of the water-soluble polymers include, for example, hydroxypropylmethyl cellulose having a molecular weight of 1,000 to 4,000,000, hydroxymethyl cellulose, hydroxyethyl cellulose, hydroxypropyl cellulose having a molecular weight of 2,000 to 2,000,000, hydroxypropylmethyl cellulose phthalate having a labeled viscosity of 30 to 200 mm²/s [at 20°C; a 10% solution prepared by dissolving hydroxypropylmethyl cellulose phthalate in a methanol/dichloromethane mixture (1:1)], carboxyvinyl polymers, chitosans, mannans, galactomannans, xanthans, carageenans, amylose, alginic acid, salts and derivatives thereof, pectin, acrylates, aminoalkylmethacrylate copolymers, methacrylate copolymers, polyacid anhydrides, polyamino acids, poly(methylvinyl ether/maleic anhydride)polymers, polyvinyl alcohols, polyvinylpyrrolidone, glucans, scleroglucans, carboxymethyl cellulose and derivatives thereof, methyl cellulose, or conventional water-soluble cellulose derivatives. Hydroxypropylmethyl cellulose having a molecular weight of 1,000 to 2,000,000, or carboxyvinyl polymers of 3,000 to 45,000 cps (at 25°C; a 0.5% aqueous solution) is preferable, and hydroxypropylmethyl cellulose having a molecular weight of 10,000 to 1,000,000, or carboxyvinyl polymers of 4,000 to 40,000 cps (at 25°C; a 0.5% aqueous solution) is more preferable. The content of the water-soluble polymer is 10 W/W% or more per formulation unit, preferably 30 W/W% or more, more preferably 70 W/W% or more. These water-soluble polymers may be contained alone or as a combination thereof in an appropriate amount(s).

Various fillers for medicaments may be appropriately used to prepare the matrix formulation of the present invention. The fillers for medicaments are not particularly limited, so long as they are pharmaceutically acceptable and may be used as additives for medicament. As the fillers, for example, a diluent, a binder, a disintegrator, an acidulant, an effervescent agent, an artificial sweetener, a flavor, a lubricant, a coloring agent, or the like may be used. The diluent may be selected from mannitol, lactose, starches derived from various organs, sorbitol, xylitol, citric acid, microcrystalline cellulose, and/or a diluent capable of generally promoting a penetration of water or an aqueous liquid into a pharmaceutical preparation. The binders include, for example, hydroxypropylmethyl cellulose, hydroxypropyl cellulose, polyvinyl alcohol, methyl cellulose, gum arabic, and the like. The disintegrators include, for example, a corn starch, a starch, carmellose calcium, carmellose sodium, low-substituted hydroxypropyl cellulose, and the like. The acidulants include, for example, citric acid, tartaric acid, malic acid, and the like. The effervescent agents include, for example, sodium bicarbonate and the like. The artificial sweeteners include, for example, saccharin sodium, dipotassium glycyrrhizinate, aspartame, stevia, thaumatin, and the like. The flavors include, for example, lemon, lemon-lime, orange, menthol, and the like. The lubricants include, for example, magnesium stearate, calcium stearate, sucrose fatty acid esters, polyethylene glycol, talc, stearic acid, and the like. These fillers for medicaments may be contained alone or as a combination thereof in an appropriate amount(s).

The matrix formulation of the present invention may be manufactured by a known method per se. In particular, tablets may be manufactured by a tablet forming method which is commonly used and known to those skilled in the art. The tabletting pressure is generally within a range of 3 to 20 kN. In a small scale, tablets may be prepared, in accordance with methods explained in detail in the following Examples, by preparing powder and/or granules with a mortar and a pestle, and forming the powder and/or granules into tablets by using an oil press tabletting machine.

### (5) Sustained release formulation with coating membrane

As a method for controlling the release (i.e., sustained release) of a drug from a pharmaceutical preparation, a coating membrane is applied to the surface of a pharmaceutical preparation by coating. The kind of coating membrane is not particularly limited. The coating may be applied to not only a shaped preparation such as a tablet or the like, but also various preparations such as powder, granules, pellets, or the like.

A coating liquid may contain, for example, a membrane forming agent (mainly a polymer), a plasticizer (which provides plasticity, flexibility, and extensibility to a coating membrane), a water-soluble base (such as lactose, sodium chloride, or the like), a dispersing agent (which prevents particles or tablets from adhering and aggregating after the coating), or the like. These components may be dissolved or dispersed in an appropriate solvent, such as water, alcohol, or the like, to prepare the coating liquid.

The release of a drug from the formulation can be controlled by appropriately adjusting, for example, the kinds and the mixing ratio of components contained in the coating liquid, the amount of coating, or the like. For example, a preferable ratio of the membrane forming agent to the water-soluble base is 99:1 to 50:50 (membrane forming agent: water-soluble base). The content of the coating membrane is preferably approximately 2 to 30 parts by weight, with respect to 100 parts by weight of an uncoated tablet.

Examples of a coating method include, for example, a method in which a coating liquid, such as an organic solvent solution, or a mixing solution or suspension of an organic solvent and water, is sprayed while being rotated, by using a coating pan, or a method in which a coating liquid is sprayed while being fluidized by air blown from the bottom of a fluidized bed. Further, a coating liquid prepared by dissolving or dispersing a membrane forming agent in a solvent may be sprayed, and then the solvent may be removed by drying to form a coating membrane on the surface of a pharmaceutical preparation. As a simple method, a coating membrane may be formed by immersing shaped preparations or the like in a coating liquid.

Examples of the membrane forming agent as used herein include, for example, a water-insoluble polymer or a water-soluble polymer. The membrane forming agent is not particularly limited, so long as it is pharmaceutically acceptable and biocompatible. These membrane forming agents may be added alone or as a combination thereof in an appropriate amount(s).

Examples of the water-insoluble polymer include, for example, dibenzyl phthalate, dihexyl phthalate, butyl octyl phthalate, beeswax, carnauba wax, cetyl alcohol, cetyl stearyl alcohol, glyceryl behenate, lipids, fats, resins such as shellac or the like, cellulose derivatives such as ethyl cellulose, cellulose acetate, or the like, polyacrylate derivatives such as aminoalkylmethacryl copolymer (product name: Eudragit RS) or the like, polymethacrylate derivatives such as methacrylate copolymer (product name: Eudragit L) or the like, hydroxypropylmethyl cellulose acetate succinate, polylactic acid, polyglycolic acid, or the like.

Examples of the water-soluble polymer include, for example, hydroxypropylmethyl cellulose, hydroxypropyl cellulose, hydroxyethyl cellulose, carmellose sodium, methyl cellulose, polyvinylpyrrolidone, polyethylene glycol, polyvinyl alcohol, or the like.

To enhance the hydrophilic property of the coating membrane, a water-soluble base may be added. Examples of the water-soluble base include, for example, maltose, sucrose, lactose, sodium chloride, citric acid, polyethylene glycol 400, dextrose, fructose, xylitol, polyoxyethylene sorbitan monooleate, or the like.

The coating liquid which may be used in the present invention preferably contains one or more of the above-mentioned water-insoluble polymers, and more preferably further contains one or more of the water-soluble polymers and/or one or more of the water-soluble bases.

Further, the coating liquid may contain a plasticizer to provide plasticity, flexibility, and extensibility to the coating membrane. Examples of the plasticizer include, for example, triacetin, dioctyl azelate, epoxidized tallate, triisooctyl trimellitate, triisononyl trimellitate, sucrose acetate isobutyrate, soybean oil, propylene glycol, glycerol, polyethylene glycol, glyceryl triacetate (triacetin), triethyl citrate, acetyl triethyl citrate, diethyl phthalate, diethyl sebacate, dibutyl sebacate, acetylated monoglyceride, castor oil, liquid paraffin, or the like.

If desired, a surfactant and/or a disintegrator may be added. As such a surfactant which may be used in the coating membrane, a surfactant having an HLB value of approximately 10 to 25, such as polyethylene glycol 400 monostearate, polyoxyethylene-4-sorbitan monolaurate, polyoxyethylene-20-sorbitan monooleate, polyoxyethylene-20-sorbitan monopalmitate, polyoxyethylene-20-monolaurate, polyoxyethylene-40-stearate, sodium oleate, or the like, may be used.
Examples of the disintegrator include, for example, starches, clay, cellulose, algin, gums, crosslinked starches, crosslinked cellulose, or crosslinked polymers. Typically, for example, corn starch, potato starch, croscarmellose, crospovidone, sodium starch glycorate, Veegum HV, methyl cellulose, agar, bentonite, carboxyl methyl cellulose, alginic acid, guar gum, or the like, may be used.

As a solvent suitable for manufacturing the formulation of the present invention, an aqueous or inert organic solvent which does not adversely affect substances used in the system may be used. Examples of the solvent include, for example, aqueous solvents, alcohols, ketones, esters, ethers, aliphatic hydrocarbons, halogenated solvents, cycloaliphatic, aromatic, or heterocyclic solvents, or a mixture thereof. Typical solvents may be, for example, acetone, diacetone alcohol, methanol, ethanol, isopropanol, butanol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloride, nitroethane, nitropropane, tetrachloroethane, ethyl ether, isopropyl ether, cyclohexane, cyclooctane, benzene, toluene, naphtha, 1,4-dioxane, tetrahydrofuran, diglyme, water, an aqueous solvent containing an inorganic salt such as sodium chloride, calcium chloride, or the like, or a mixture thereof, such as a mixture of acetone and water, a mixture of acetone and methanol, a mixture of acetone and ethanol, a mixture of methylene dichloride and methanol, or a mixture of ethylene dichloride and methanol.

### (6) Multi-layered formulation consisting of drug core and release-controlling layer which are geometrically arranged

A multilayered formulation, an embodiment of the sustained release pharmaceutical composition for tacrolimus according to the present invention, may be a two-layered or three-layered sustained release formulation, characterized by consisting of a drug-containing layer and a release-controlling layer, and consisting of:
a) the first layer (layer 1) which is prepared by compressing a mixture or granules containing 5 to 90 W/W% (preferably 10 to 85 W/W%) of a water-soluble polymer in this layer, and has a property of being swollen by contact with environmental fluids,
b) the second layer (layer 2) comprising tacrolimus or a pharmaceutically acceptable salt thereof, a water-soluble polymer, and other filler(s), which is adjacent to the first layer, has a property suitable to compression-molding, and is designed to release the physiologically active substance within a predetermined period of time, and
c) the third layer (layer 3) (which may be optionally adjacent to the layer 2) which contains a water-soluble polymer capable of being generally gelled and/or swollen followed by optionally being disintegrated, and has a property of controlling the release of tacrolimus or a pharmaceutically acceptable salt thereof from the layer 2. The "environmental fluids" include, for example, an aqueous solution as used in a dissolution test, as well as body fluids such as blood or gastrointestinal fluids.

Techniques for such a multilayered formulation which may be used in the sustained release pharmaceutical composition for tacrolimus according to the present invention are disclosed in, for example, U.S. Patent No. 4,839,177, U.S. Patent No. 5,422,123, U.S. Patent No. 5,780,057, U.S. Patent No. 6,149,940, Japanese Patent Publication (Kokai) No. 2005-162736, and Japanese Patent Publication (Kokai) No. 2005-162737, the contents of which are incorporated herein by reference. As disclosed in U.S. Patent No. 4,839,177 and U.S. Patent No. 5,422,123, the multilayered formulation is **characterized in that** a release rate of the drug from the pharmaceutical formulation is controlled by sandwiching the layer 2 containing the drug between the layer 1 and the layer 3 in which the drug is not contained or is optionally contained. Further, as disclosed in U.S. Patent No. 5,780,057 and U.S. Patent No. 6,149,940, it is known that when the multilayered formulation is brought into contact with body fluids, at least one of the layer 1 and the layer 3 are rapidly swollen followed by the layer 2 is swollen, that is, the volume of the formulation is significantly increased, and as a result, the formulation remains in the stomach for a longer period of time, and almost all of the active substance contained therein is released and absorbed at the upper gastrointestinal tract in a controlled manner.

The layer 1 and the layer 3 may have the same composition and the same functional properties, or may have different compositions and different functional properties. When the layer 1 and the layer 3 have the same composition and functional properties, the amounts and thicknesses of the layers 1 and 3 which sandwich the layer 2 may be changed. At least one of the layers 1 and 3 acts as a barrier for the release of the active substance, that is, it is impermeable enough for tacrolimus or a pharmaceutically acceptable salt thereof contained in the layer 2 not to be released or diffused therefrom. Further, at least one of the layers 1 and 3 can be rapidly swollen, that is, the volume thereof is rapidly increased. The layer 3 may optionally contain the drug so that a drug release which is different from that released from the layer 2 can be supplementally added to the pharmaceutical formulation.

The water-soluble polymers used in the layer 1, the layer 3, and the layer 2 are not particularly limited, so long as they are pharmaceutically acceptable and biocompatible. Such water-soluble polymers may be gradually dissolved and/or gelled, and/or may be gelled rapidly or at a different rate and then optionally disintegrated in an aqueous liquid. Examples of the water-soluble polymers include, for example, hydroxymethyl cellulose, hydroxyethyl cellulose, hydroxypropylmethyl cellulose having a molecular weight of 1,000 to 4,000,000, hydroxypropyl cellulose having a molecular weight of 2,000 to 2,000,000, carboxyvinyl polymers, chitosans, mannans, galactomannans, xanthans, carageenans, amylose, alginic acid, salts and derivatives thereof, pectin, acrylates, methacrylates, acrylate/methacrylate copolymers, polyacid anhydrides, polyamino acids, poly(methylvinyl ether/maleic anhydride)polymers, polyvinyl alcohols, glucans, scleroglucans, carboxymethyl cellulose and derivatives thereof, ethyl cellulose, methyl cellulose, or conventional water-soluble cellulose derivatives. Hydroxypropylmethyl cellulose having a molecular weight of 3,000 to 2,000,000 is preferable. The content of the water-soluble polymer in the layer 1 or the layer 3 is generally 5 to 90 W/W%, preferably 10 to 85 W/W%, more preferably 20 to 80 W/W%, with respect to the weight of each layer. The content of the water-soluble polymer in the layer 2 is generally 5 to 90 W/W%, preferably 10 to 85 W/W%, to the weight of the layer.

In the process for preparing the layer 1 and the layer 3, a water-soluble filler which promotes the degree of wetness of the layers may be used, to rapidly increase the volume of the multilayerd formulation containing the above water-soluble polymer. The water-soluble filler may be preferably selected from a group of fillers having an extremely rapid disintegrability, such as cross-linked polyvinylpyrrolidone, hydroxypropyl cellulose or hydroxypropylmethyl cellulose having a low or medium molecular weight, cross-linked carboxymethyl cellulose sodium, carboxymethyl starch or salts thereof, divinylbenzene/potassium methacrylate copolymers, or the like.
The content of the filler is 1 to 90 W/W% or less, preferably 5 to 50 W/W% of each layer.

If desired, a surfactant (anionic, cationic, or nonionic surfactants) may be further used to improve the degree of wetness. As a result, tablets and environmental fluids may conform with each other more rapidly, and the tablets, particularly the gel-forming layer, may be gelled more rapidly. Examples of the surfactant include, for example, sodium laurylsulfate, sodium ricinolate, sodium tetradecylsulfonate, sodium dioctylsulfosuccinate, cetomagrogol, poloxamer, glycerol monostearate, polysorbate, sorbitan monolaurate, lecithins, or other pharmaceutically acceptable surfactants.

If desired, another substance which modifies hydration may be further used. Such a substance may be selected from, for example, mannitol, lactose, starches derived from various organs, sorbitol, xylitol, microcrystalline cellulose, and/or a diluent capable of generally promoting a penetration of water or an aqueous liquid into a pharmaceutical composition; or a hydrophobic diluent to retard a penetration of water or an aqueous liquid into a pharmaceutical formulation, such as ethyl cellulose, glycerol monostearate, palmitate, or hydrogenated or non-hydrogenated vegetable oils (for example, hydrogenated castor oil, wax, monoglyceride, diglyceride, or triglyceride). It is preferable to select ethyl cellulose or hydrogenated vegetable oils as the hydrophobic diluent.

The content of the hydrophobic diluent in the layer 1 or the layer 3 is generally 1 to 60 W/W%, preferably 5 to 40 W/W%, more preferably 10 to 30 W/W%, with respect to the weight of each layer.
To control the release rate of tacrolimus from the pharmaceutical formulation, microcrystalline cellulose or a water-soluble base, such as dextrose, sucrose, fructose, maltose, xylitol, citric acid, lactose, mannitol, or the like, may be used in the layer 2, if desired.
The content of microcrystalline cellulose and/or the water-soluble base in the layer 2 is generally 5 to 90 W/W%, preferably 10 to 80 W/W%, more preferably 20 to 70 W/W%, with respect to the weight of the layer.

The multilayered formulation of the present invention may contain, for example, a lubricant, such as magnesium stearate, talc, stearic acid, glycerol monostearate, polyoxyethylene glycol having a molecular weight of 400 to 7,000,000, hydrogenated castor oil, glycerol behenate, monoglyceride, diglyceride, triglyceride, or the like, a fluidizing agent such as colloidal silica or other silica, a binder, a buffer, an absorbing agent, or other pharmaceutically acceptable additives.

The multilayered formulation of the present invention may be manufactured, for example, by mixing powder and/or granules by a known manufacturing technique per se, and forming the mixture into tablets by compression. A two-layered or three-layered pharmaceutical formulation, such as a tablet, may be manufactured by a known method per se. The multilayered formulation of the present invention may be manufactured, for example, by using a rotary press capable of manufacturing multilayered tablets. It is preferable that a tabletting pressure is generally 7 to 50 kN. When the tablets are manufactured on a small scale, a mortar and pestle may be used to prepare powder and/or granules, and then, an oil press tabletting machine may be used to manufacture two-layered or three-layered tablets. The thickness of each layer of the formulation may vary according to the content of the active substance, and is preferably 0.2 to 8 mm, more preferably 1 to 4 mm. In the formulation of the present invention, for example, a coating layer with a macromolecular material may be applied to the pharmaceutical composition. Such a coating may be applied by using an organic or aqueous solution, in accordance with a known method per se.

When the multilayered formulation of the present invention is brought into contact with gastric juices in the gastrointestinal tract and/or liquids, the volume thereof is rapidly increased. This increase in volume may be limited in a single layer or several layers of the formulation. Such a formulation may be in a form of a tablet, small tablets, or a gelatin capsule consisting of small tablets. Further, at least two small tablets may be combined in the same formulation, and may be packed in, for example, a wafer capsule or a gelatin capsule. When the formulation consists of small tablets, each small tablet may have a different composition or the same composition.

### (7) Gel formulation in which a plurality of gums is combined

A gel formulation in which a plurality of gums is combined, an embodiment of the sustained release pharmaceutical composition for tacrolimus according to the present invention, is characterized by comprising at least the solid dispersion of tacrolimus and a gum base. The gum base as used herein means a sustained release filler comprising a homopolysaccharide which can form a crosslinkage with a heteropolysaccharide gum when exposed to the heteropolysaccharide gum and environmental fluids (such as body fluids, an aqueous solution for an in vitro dissolution test, or the like). The sustained release filler may further comprise calcium sulfate and/or a water-soluble base. The gel formulation may further contain a commonly used filler.

Techniques for obtaining the gel formulation in which a plurality of gums is combined, which may be used in the sustained release pharmaceutical composition for tacrolimus according to the present invention, are disclosed in, for example, U.S. Patent No. 4,994,276, U.S. Patent No. 5,128,143, U.S. Patent No. 5,135,757, and Japanese Patent No. 2832248. As disclosed therein, it is known that a heterogeneously dispersed filler comprising a combination of a heteropolysaccharide and a homopolysaccharide exhibiting a synergistic effect, such as a combination of two or more polysaccharide gums, has a viscosity higher than that of any single gum, and can cause a rapid hydration, and thus a harder gel is generated more rapidly and rigidly. The contents of the above patent references are incorporated herein by reference.

The heteropolysaccharide as used herein is defined as a water-soluble polysaccharide containing two or more sugar units. The heteropolysaccharide is not particularly limited, so long as it has a branched-chain or spiral configuration, and has an excellent water absorbing property and a high viscosity improving property. As the heteropolysaccharide, for example, xanthan gum or derivatives thereof (such as deacylated xanthan gum), carboxymethyl ether, or propylene glycol ester are preferable, and xanthan gum having a high molecular weight (>10⁶) is more preferable.

The homopolysaccharide as used herein is not particularly limited, so long as it is a polysaccharide consisting of mannose and galactose, and can form a crosslinkage with a heteropolysaccharide. Locust bean gum having a high ratio of mannose to galactose is more preferable than other galactomannans such as guar or hydroxypropyl guar.
Other naturally-occurring polysaccharide gums may be used in the present invention. Examples of such polysaccharides include, for example, alginic acid derivatives, carrageenan, tragacanth gum, gum arabic, karaya gum, polyethylene glycol esters of these gums, chitin, chitosan, mucopolysaccharide, konjak, starch, substituted starch, starch fragment, dextrin, British gum having a molecular weight of approximately 10,000 Da, dextran, or the like. The starch may be used in an unmodified form, for example, an ungelled starch such as potato, rice, banana, or the like, or a semisynthetic or gelled starch.

As a combination of the heteropolysaccharide and the homopolysaccharide, the combination of xanthan gum and locust bean gum is particularly preferable. The content ratio of the heteropolysaccharide and the homopolysaccharide is not particularly limited, so long as it is an amount effective in enhancing a desired gel strength. Such a ratio (heteropolysaccharide gum: homopolysaccharide gum) is approximately 3:1 to approximately 1:3, preferably approximately 1:1.

The water-soluble cationic crosslinking agent as used herein is not particularly limited, so long as it is a pharmaceutically acceptable monovalent or polyvalent metal cation. As the binder, for example, calcium sulfate or the like may be used.

The water-soluble base as used herein is not particularly limited, so long as it is pharmaceutically acceptable. Examples of the water-soluble base include, for example, dextrose, sucrose, fructose, maltose, xylitol, citric acid, or the like.

The gel formulation in which a plurality of gums is combined of the present invention may be manufactured, for example, in a pharmaceutically acceptable form for oral administration such as a tablet or the like. In an embodiment, (1) a heteropolysaccharide gum, and a homopolysaccharide which can form a crosslinkage with the heteropolysaccharide gum when exposed to environmental fluids are mixed together under the dry condition with a pharmaceutically acceptable water-soluble base in a desired ratio, (2) the resulting mixture is subject to a wet granulation, (3) the granules are dried, (4) the dried granules are pulverized to obtain a sustained release filler having a desired particle size, (5) the resulting sustained release filler is granulated together with tacrolimus or a pharmaceutical acceptable salt thereof, (6) the resulting granules are dried, (7) a conventional filler, such as a lubricant or the like, is added thereto, and (8) the resulting mixture is formed by compression into, for example, tablets. In another embodiment, a mixture of the sustained release filler and tacrolimus or a pharmaceutical acceptable salt thereof may be granulated, together with an a solution of a hydrophobic substance (such as ethyl cellulose or the like) in an amount sufficient to retard the hydration of the filler (i.e., gums) without the destruction thereof, and then a conventional filler such as a lubricant is added thereto, and the resulting mixture is formed by compression into, for example, tablets.

In the wet granulation, predetermined amounts of the heteropolysaccharide gum, the homopolysaccharide gum, the cationic crosslinking agent, and the water-soluble base are homogeneously mixed; and then, a wetting agent, such as water, propylene glycol, glycerol, alcohol, or the like, is added thereto to prepare a wet aggregate; and the resulting wet aggregate is dried, and pulverized using a conventional apparatus to prepare granules having a predetermined particle size.

As the lubricant, for example, stearic acid or the like may be used. The mixing of the hydrophobic substance with the sustained release filler may be carried out, for example, by using a liquid in which the hydrophobic substance is dissolved and/or dispersed in an organic solvent, and further granulating the above-mentioned granules together with the liquid.
Examples of the hydrophobic substance include, for example, a pharmaceutical acceptable hydrophobic cellulose, such as ethyl cellulose or the like.

A combination and a mixing ratio of each component are not particularly limited. In a preferred embodiment, approximately 5 to 60 W/W% of xanthan gum (as the heteropolysaccharide) and locust bean gum (as the homopolysaccharide) (xanthan gum: locust bean gum = approximately 1:1) with respect to the total weight of the pharmaceutical formulation may be contained, and approximately 10 W/W% or less of calcium sulfate (as the water-soluble cationic crosslinking agent) and approximately 30 to 70 W/W% of dextrose (as an inert diluent) may be further contained. To control the release rate, the hydrophobic substance may be added, and, for example, approximately 5 to 10 W/W% of ethyl cellulose may be contained.

### EXAMPLES

The present invention will now be further illustrated by, but is by no means limited to, the following Examples.

### Example 1: Preparation of sustained release hydrogel-forming formulation containing tacrolimus

One part of tacrolimus was dissolved in 5 mL of ethanol in a mortar. One part of hydroxypropylmethyl cellulose was added thereto and mixed well with a pestle. Further, 2.5 mL of dichloromethane was added and mixed well until the whole was dissolved. Then, 1 part of croscarmellose sodium and 2 parts of lactose were further added and mixed well with the pestle in the mortar. The mixture was dried by evaporation until the solvents were completely removed, to obtain a solid dispersion of tacrolimus (hereinafter referred to as solid dispersion 1). In accordance with the formulations shown in Table 1, 5 mg of solid dispersion 1 (corresponding to 1 mg of tacrolimus), polyethylene glycol (PEG) 6000 (manufactured by Sanyo Chemical Industries, Ltd.), and polyethylene oxide (Polyox WSR303, manufactured by The Dow Chemical Company) were added and mixed well using a pestle and a mortar. Each mixed powder (165 mg) was compression-molded by using an oil press tabletting machine (tabletting pressure = 1000 kg/punch) to obtain sustained release tacrolimus formulations (1A and 1B) having a diameter of 7 mm according to the present invention.

### Test Example 1: Dissolution test

Drug-releasing properties of formulations 1A and 1B prepared in Example 1 were evaluated by a dissolution test, method 2 (paddle method), described in the Japanese Pharmacopoeia. As a test medium, 900 mL of a solution prepared by dissolving 0.005% of hydroxypropylmethyl cellulose (HPC:HPC-M) in a phosphate buffer (pH4.5) was used, and the test was carried out at a paddle rotation speed of 100 rpm without the use of a sinker. Samples were collected at predetermined times, and amounts of tacrolimus in the sampling solutions were measured using an HPLC with an ultraviolet and visible detector (a detecting wavelength = 210 nm).

### Results:

Dissolution rates of formulations 1A and 1B after 4 hours from the beginning of the dissolution test were 12% and 23%, respectively. Further, dissolution rates of formulations 1A and 1B after 24 hours from the beginning of the dissolution test were 84% and 88%, respectively.

**[Table 1]**

| Components (unit: mg) | 1A | 1B |
|---|---|---|
| Solid dispersion 1 | 5 | 5 |
| Polyox WSR303 | 80 | 40 |
| PEG 6000 | 80 | 120 |
| Total weight | 165 | 165 |
| Tablet size (mm) | 7 × 7R | 7 × 7R |
| Dissolution rate after 4 hours (%) | 12 | 23 |
| Dissolution rate after 24 hours (%) | 84 | 88 |

### Example 2: Preparation of sustained release hydrogel-forming formulation containing tacrolimus

In a mortar, 1 g of Eudragit EPO (degussa; powder product of Eudragit E) was dissolved in 3 mL of methanol. Further, 200 mg of tacrolimus was added thereto, stirred with a pestle, and mixed well until the whole was dissolved. The mixture was mixed by stirring until the solvent was completely removed, and dried by evaporation, to obtain a solid dispersion of tacrolimus (solid dispersion 2). In accordance with the formulations shown in Table 2, 6 mg of solid dispersion 2 (containing the water-insoluble base and the equivalent corresponding to 1 mg of tacrolimus), PEG 6000, and Polyox WSR303 are added and mixed well using a pestle and a mortar. Each mixed powder (166 mg) is compression-molded by using an oil press tabletting machine (tabletting pressure = 1 t/tablet) to obtain sustained release tacrolimus formulations (2A and 2B) having a diameter of 7 mm according to the present invention.

**[Table 2]**

| Components (unit: mg) | 2A | 2B |
|---|---|---|
| Solid dispersion 2 | 6 | 6 |
| Polyox WSR303 | 80 | 40 |
| PEG6000 | 80 | 120 |
| Total weight | 166 | 166 |
| Tablet size (mm) | 7 × 7R | 7× 7R |

### Comparative Example 1

One part of tacrolimus was dissolved in ethanol in a mortar. To the mortar, 0.3 part of ethylcellulose was added and mixed well until the whole was dissolved. Further, 0.3 part of hydroxypropylmethyl cellulose (HPMC 2910) and 2 parts of lactose were added thereto, and mixed well until the solvents were completely removed, to obtain a solid dispersion of tacrolimus (solid dispersion 3). To 3.6 mg of solid dispersion 3 (corresponding to 1 mg of tacrolimus), 105.3 mg of lactose and 1.1 mg of magnesium stearate were added and mixed well. Gelatin capsules were filled with this mixed powder (110 mg) to obtain a sustained release tacrolimus formulation (R).

### Test Example 2: Dissolution test

A drug-releasing property of formulation R prepared in Comparative Example 1 was evaluated by a dissolution test, method 2 (paddle method), described in the Japanese Pharmacopoeia. The evaluation was carried out in a similar fashion as shown in Test Example 1, except that a sinker was used.

### Results:

Dissolution rates of formulation R after 4 hours and 24 hours from the beginning of the dissolution test were approximately 50% and approximately 80%, respectively.

### Example 3: Multi-layered formulation consisting of tacrolimus core and release-controlling layer which are geometrically arranged

### (1) Preparation of granules (A1) which form layer 1 and layer 3 (not containing drug) used in controlling the release of drug

Granules (A1) consisting of the formulation unit shown in Table 3 were prepared, and used in preparing the layer 1 and the layer 3 as the top and bottom layers of a three-layered tablet.
In accordance with the formulation shown in Table 3, hydroxypropylmethyl cellulose (HPMC 90SH-15000; Shin-Etsu Chemical Co., Ltd.), hydrogenated caster oil, yellow ferric oxide, and magnesium stearate (St-Mg) were weighed out, and mixed well by using a mortar and a pestle until the whole was homogeneously mixed. The resulting homogeneous powder mixture was moistened with an alcohol solution containing 10%(W/V) of ethyl cellulose. The resulting homogeneously wet aggregate was dried at 40°C, and sieved through a screen to obtain granules (A1).

**[Table 3]**

| Components (unit:mg) | A1 |
|---|---|
| HPMC | 80.25 |
| Hydrogenated caster oil | 13.5 |
| Yellow ferric oxide | 0.25 |
| Ethyl cellulose | 5 |
| Magnesium stearate | 1 |
| Total | 100.00 |

### (2) Preparation of mixed powder (B1) which forms layer 2 containing active substance

A mixed powder (B1) containing a solid dispersion of tacrolimus and the formulation unit as prepared in the following procedure was prepared, and used in preparing the layer 2 as the intermediate layer of a three-layered tablet.
In accordance with the formulation shown in Table 4, mannitol, microcrystalline cellulose, hydroxypropylmethyl cellulose (HPMC 90SH-15000; Shin-Etsu Chemical Co., Ltd.), and polyvinylpyrrolidone were weighed out, and mixed well by using a mortar and a pestle until the whole was homogeneously mixed, to prepare a B1 intermediate powder. To 195 mg of the resulting B1 intermediate powder, 5 mg of tacrolimus solid dispersion 1 prepared in a similar fashion as shown in Example 1 was added, and the whole was homogeneously mixed well by using a mortar and a pestle, to obtain a tacrolimus-containing mixed powder (B1) used as the layer 2 which was the intermediate layer of a three-layered tablet.

**[Table 4]**

| Components (unit: mg) | B1 intermediate powder |
|---|---|
| Lactose | 11 |
| Mannitol | 20 |
| HPMC | 20 |
| Polyvinylpyrrolidone | 6.4 |
| Microcrystalline cellulose | 137.6 |
| Total | 195.0 |

### (3) Preparation of three-layered tablet (compression molding)

Three-layered tablets were prepared by using an oil press tabletting machine. The tabletting was carried out using a punch having a diameter of 8.0 mm × 8.0 mmR under a tabletting pressure of 1000 kg/punch.
As the layer 3, 150 mg of the granules (A1) prepared in Example 3(1) were put into a die, and a tapping was carried out to flatten the upper surface. As the layer 2, 100 mg of the mixed powder (B1) containing the active substance prepared in Example 3(2) was further loaded onto the layer 3 in the die, and a tapping was carried out to flatten the upper surface. Furthermore, as the layer 1, 100 mg of the granules (A1) prepared in Example 3(1) were loaded onto the layer 2. Compression-molding (1000 kg/punch; holding for 10 seconds) was carried out to obtain a sustained release tacrolimus formulation (3) of the present invention, as a three-layered tablet having a tablet weight of 350 mg and containing 1 mg of tacrolimus. The compression-molding may be carried out by loading the layers 1 and 3 into the die in inverse order.

### Example 4: Multi-layered formulation consisting of tacrolimus core and release-controlling layer which are geometrically arranged

### (1) Preparation of mixed powder (B2) which forms layer 2 containing active substance

To 180 mg of the B1 intermediate powder prepared in Example 3(2), 10 mg of hydroxypropylmethyl cellulose (HPMC 90SH-15000; Shin-Etsu Chemical Co., Ltd.) and 5 mg of tacrolimus solid dispersion 1 prepared in a similar fashion as shown in Example 1 were added, and the whole was homogeneously mixed well by using a mortar and a pestle, to obtain a tacrolimus-containing mixed powder (B2) used as the layer 2 which was the intermediate layer of a three-layered tablet.

### (2) Preparation of three-layered tablet (compression molding)

Three-layered tablets were prepared by using an oil press tabletting machine. The tabletting was carried out using a punch having a diameter of 7.0 mm ×8.4 mmR under a tabletting pressure of 1000 kg/punch.
As the layer 3, 50 mg of the granules (A1) prepared in Example 3(1) were put into a die, and a tapping was carried out to flatten the upper surface. As the layer 2, 195 mg of the mixed powder (B2) containing the active substance prepared in Example 4(1) was further loaded onto the layer 3 in the die, and a tapping was carried out to flatten the upper surface. Furthermore, as the layer 1, 50 mg of the granules (A1) prepared in Example 3(1) were loaded onto the layer 2. Compression-molding (1000 kg/punch; holding for 10 seconds) was carried out to obtain a sustained release tacrolimus formulation (4) of the present invention, as a three-layered tablet having a tablet weight of 295 mg and containing 1 mg of tacrolimus.

### Example 5: Multi-layered formulation consisting of tacrolimus core and release-controlling layer which are geometrically arranged

Three-layered tablets were prepared by using an oil press tabletting machine. The tabletting was carried out using a punch having a diameter of 8.0 mm ×8.0 mmR under a tabletting pressure of 1000 kg/punch.
As the layer 3, 150 mg of the granules (A1) prepared in Example 3(1) were put into a die, and a tapping was carried out to flatten the upper surface. As the layer 2, 195 mg of the mixed powder (B2) containing the active substance prepared in Example 4(1) was further loaded onto the layer 3 in the die, and a tapping was carried out to flatten the upper surface. Furthermore, as the layer 1, 100 mg of the granules (A1) prepared in Example 3(1) were loaded onto the layer 2. Compression-molding (1000 kg/punch; holding for 10 seconds) was carried out to obtain a sustained release tacrolimus formulation (5) of the present invention, as a three-layered tablet having a tablet weight of 445 mg and containing 1 mg of tacrolimus.

### Test Example 3: Dissolution test

Drug-releasing properties of formulations 3 to 5 prepared in Examples 3 to 5 were evaluated by a dissolution test, method 2 (paddle method), described in the Japanese Pharmacopoeia. As a test medium, 900 mL of a solution prepared by dissolving 0.005% of hydroxypropylmethyl cellulose (HPC:HPC-M) in a second fluid (JP2) of a disintegration test described in the Japanese Pharmacopoeia was used, and the test was carried out at a paddle rotation speed of 100 rpm without the use of a sinker. Samples were collected at predetermined times, and amounts of tacrolimus in the sampling solutions were measured using an HPLC with an ultraviolet and visible detector (a detecting wavelength = 210 nm).

### Results:

Dissolution rates of formulations 3 to 5 after 4 hours and 24 hours from the beginning of the dissolution test are shown in Table 5. The dissolution rates of formulations 3 to 5 were 7.3% to 28.5% after 4 hours from the beginning of the dissolution test, and 71.6% or more after 24 hours therefrom.

**[Table 5]**

| | Example 3 | Example 4 | Example 5 |
|---|---|---|---|
| Dissolution rate after 4 hours (%) | 28.5 | 13.2 | 7.3 |
| Dissolution rate after 24 hours (%) | >87.7 | 74.5 | 71.6 |

### Example 6: Multi-layered formulation consisting of tacrolimus core and release-controlling layer which are geometrically arranged: Preparation of three-layered tablet containing water-insoluble base in layer 2 (compression molding)

A mixed powder used as the layer 2 is prepared in accordance with the procedures described in Example 3(2) and Example 4(1), except that 6 mg of tacrolimus solid dispersion 2 (corresponding to 1 mg of tacrolimus) which is prepared in a similar fashion as shown in Example 2 is used as a tacrolimus solid dispersion. A sustained release tacrolimus formulation, as a three-layered tablet in which a water-insoluble base is contained in the layer 2, is prepared in accordance with the procedures described in Example 3(3), Example 4(2) and Example 5, except that the obtained mixed powder is used.

### Example 7: Gel formulation in which a plurality of gums is combined

Five parts of locust bean gum, 5 parts of xanthan gum, 7 parts of dextrose, and 1 part of calcium sulfate were weighed out, and mixed well by using a mortar and pestle until the whole was homogeneously mixed, to prepare a mixed powder. Then, 2 mL of purified water was divided into two aliquots, and these aliquots were dropwisely added to the resulting mixed powder (1 mL × twice). The whole was mixed well by stirring using a pestle, to form granules. The resulting granules were sieved through a 16 mesh (0.59 µm) screen, and dried at a constant temperature of 40°C for 12 hours to obtain a granulated powder (C1).

In accordance with the formulations shown in Table 6, the granulated powder (C1) was mixed with 5 mg of solid dispersion 1 (corresponding to 1 mg of tacrolimus) prepared in a similar fashion as shown in Example 1. The whole was mixed well by stirring using a mortar and a pestle. A die was filled with the resulting mixture, and compression-molding was carried out under tabletting conditions shown in Table 6 by using an oil press tabletting machine, to obtain sustained release tacrolimus formulations (7A to 7C) of the present invention.
Further, 180 mg of the granulated powder (C1) was mixed with 5 mg of solid dispersion 1 (corresponding to 1 mg of tacrolimus) prepared in a similar fashion as shown in Example 1, followed by dextrose. The whole was mixed well by stirring using a mortar and a pestle. A die was filled with the resulting mixture, and compression-molding was carried out under tabletting conditions shown in Table 6 by using an oil press tabletting machine, to obtain sustained release tacrolimus formulations (7D to 7F) of the present invention.

In accordance with the formulations shown in Table 7, the granulated powder (C1) is mixed with 6 mg of solid dispersion 2 (corresponding to 1 mg of tacrolimus) which is prepared in a similar fashion as shown in Example 2. The whole is mixed well by stirring using a mortar and a pestle. A die is filled with the resulting mixture, and compression-molding is carried out under tabletting conditions shown in Table 7 by using an oil press tabletting machine, to obtain sustained release tacrolimus formulations (7G to 7I) of the present invention.
Further, 180 mg of the granulated powder (C1) is mixed with 6 mg of solid dispersion 2 (corresponding to 1 mg of tacrolimus) which is prepared in a similar fashion as shown in Example 2, followed by dextrose. The whole is mixed well by stirring using a mortar and a pestle. A die is filled with the resulting mixture, and compression-molding is carried out under tabletting conditions shown in Table 7 by using an oil press tabletting machine, to obtain sustained release tacrolimus formulations (7J to 7L) of the present invention.

**[Table 6]**

| Components (unit:mg) | 7A | 7B | 7C | 7D | 7E | 7F |
|---|---|---|---|---|---|---|
| Solid dispersion 1 | 5 | 5 | 5 | 5 | 5 | 5 |
| Granulated powder (C1) | 60 | 90 | 180 | 180 | 180 | 180 |
| Dextrose | - | - | - | 50 | 100 | 150 |
| Total weight (mg) | 65 | 95 | 185 | 235 | 285 | 335 |
| Size (mm) | 5×6R | 6.5×6.5R | 8×8R | 8×8R | 8.5×8.5R | 9×9R |
| Tabletting pressure (kg/punch) | 250 | 250 | 1000 | 500 | 500 | 500 |

**[Table 7]**

| Components (unit:mg) | 7G | 7H | 7I | 7J | 7K | 7L |
|---|---|---|---|---|---|---|
| Solid dispersion 2 | 6 | 6 | 6 | 6 | 6 | 6 |
| Granulated powder (C1) | 60 | 90 | 180 | 180 | 180 | 180 |
| Dextrose | - | - | - | 50 | 100 | 150 |
| Total weight (mg) | 66 | 96 | 186 | 236 | 286 | 336 |
| Size (mm) | 5×6R | 6.5×6.5R | 8×8R | 8×8R | 8.5×8.5R | 9×9R |
| Tabletting pressure (kg/punch) | 250 | 250 | 1000 | 500 | 500 | 500 |

### Test Example 4: Dissolution test

Drug-releasing properties of formulations 7A to 7F prepared in Example 7 were evaluated by the method described in Test Example 3.

### Results:

Dissolution rates of formulations 7A to 7F after 4 hours and 24 hours from the beginning of the dissolution test are shown in Table 8. The dissolution rates of formulations 7A to 7F were 8% to 23% after 4 hours from the beginning of the dissolution test, and 77% or more after 24 hours therefrom.

**[Table 8]**

| Examples | 7A | 7B | 7C | 7D | 7E | 7F |
|---|---|---|---|---|---|---|
| Dissolution rate after 4 hours (%) | 21 | 22 | 8 | 10 | 17 | 23 |
| Dissolution rate after 24 hours (%) | 83 | 77 | 82 | 84 | 89 | >95 |

### Example 8: Formulation utilizing swelling polymer

In accordance with the formulations shown in Table 9, 5 mg of solid dispersion 1 (corresponding to 1 mg of tacrolimus) prepared in a similar fashion as shown in Example 1 is mixed with 7.2 mg of sucrose, a predetermined amount(s) of polyethylene oxide (Polyox) and/or hydroxypropylmethyl cellulose (HPMC), and 2 mg of magnesium stearate. The whole is mixed well by stirring using a mortar and a pestle. A die is filled with the resulting mixture, and compression-molding is carried out in accordance with weights and tablet sizes shown in Table 9 by using an oil press tabletting machine, to obtain sustained release tacrolimus formulations (8A to 8F) of the present invention.

In accordance with the formulations shown in Table 10, 6 mg of solid dispersion 2 (corresponding to 1 mg of tacrolimus) which is prepared in a similar fashion as shown in Example 2 is mixed with 7.2 mg of sucrose, a predetermined amount(s) of polyethylene oxide (Polyox) and/or hydroxypropylmethyl cellulose (HPMC), and 2 mg of stearic acid. The whole is mixed well by stirring using a mortar and a pestle. A die is filled with the resulting mixture, and compression-molding is carried out in accordance with weights and tablet sizes shown in Table 10 by using an oil press tabletting machine, to obtain sustained release tacrolimus formulations (8G to 8L) of the present invention.

**[Table 9]**

| Components (unit: mg) | 8A | 8B | 8C | 8D | 8E | 8F |
|---|---|---|---|---|---|---|
| Solid dispersion 1 | 5 | 5 | 5 | 5 | 5 | 5 |
| Sucrose | 7.2 | 7.2 | 7.2 | 7.2 | 7.2 | 7.2 |
| Polyox N60K | 266 | | | | | |
| Polyox 303 | | 133 | 266 | 133 | 133 | 133 |
| Polyox 1105 | | | | 133 | | |
| HPMC (TC5E) | | | | | 133 | |
| HPMC (90SH-100,000) | | | | | | 133 |
| Magnesium stearate | 2 | 2 | 2 | 2 | 2 | 2 |
| Total weight (mg) | 280.2 | 147.2 | 280.2 | 280.2 | 280.2 | 280.2 |
| Tablet size (mm) | 7×8.4R | 6×6R | 7×8.4R | 7×8.4R | 7×8.4R | 7×8.4R |

**[Table 10]**

| Components (unit: mg) | 8G | 8H | 8I | 8J | 8K | 8L |
|---|---|---|---|---|---|---|
| Solid dispersion 1 | 6 | 6 | 6 | 6 | 6 | 6 |
| Sucrose | 7.2 | 7.2 | 7.2 | 7.2 | 7.2 | 7.2 |
| Polyox N60K | 266 | | | | | |
| Polyox 303 | | 133 | 266 | 133 | 133 | 133 |
| Polyox 1105 | | | | 133 | | |
| HPMC (TC5E) | | | | | 133 | |
| HPMC (90SH-100,000) | | | | | | 133 |
| Magnesium stearate | 2 | 2 | 2 | 2 | 2 | 2 |
| Total weight (mg) | 281.2 | 148.2 | 281.2 | 281.2 | 281.2 | 281.2 |
| Tablet size (mm) | 7×8.4R | 6×6R | 7×8.4R | 7×8.4R | 7×8.4R | 7×8.4R |

### Example 9: Matrix formulation utilizing water-soluble polymer (HPMC matrix)

In accordance with the formulations shown in Table 11, 5 mg of solid dispersion 1 (corresponding to 1 mg of tacrolimus) prepared in a similar fashion as shown in Example 1 was well mixed with hydroxypropylmethyl cellulose (HPMC), and compression-molded by using an oil press tabletting machine (tabletting pressure = 1000 kg/punch), to obtain sustained release tacrolimus formulations (9A to 9F) of the present invention.
As HPMC, TC5S (Shin-Etsu Chemical Co., Ltd.), 60SH50 (Shin-Etsu Chemical Co., Ltd.), and 60SH400 (Shin-Etsu Chemical Co., Ltd.) were used.

**[Table 11]**

| Components (unit: mg) | 9A | 9B | 9C |
|---|---|---|---|
| Solid dispersion 1 | 5 | 5 | 5 |
| HPMC (TC5S) | 395 | 345 | 295 |
| Total weight (mg) | 400 | 350 | 300 |
| Tablet size (mm) | 9.5×9.5R | 9×9R | 8.5×8.5R |
| | | | |

| Components (unit: mg) | 9D | 9E | 9F |
|---|---|---|---|
| Solid dispersion 1 | 5 | 5 | 5 |
| Mixture of lactose and magnesium stearate | 60 | 60 | 60 |
| HPMC (60SH50) | 250 | 200 | - |
| HPMC (60SH400) | - | - | 150 |
| Total weight (mg) | 315 | 265 | 215 |
| Tablet size (mm) | 9×9R | 8.5×8.5R | 8.5×8.5R |

### Test Example 5: Dissolution test

Drug-releasing properties of formulations 9A to 9F prepared in Example 9 were evaluated by the method described in Test Example 3.

### Results:

Dissolution rates of formulations 9A to 9F after 4 hours and 24 hours from the beginning of the dissolution test are shown in Table 12. The dissolution rates of formulations 9A to 9F were 16% to 32% after 4 hours from the beginning of the dissolution test, and 61% or more after 24 hours therefrom.

**[Table 12]**

| Examples | 9A | 9B | 9C | 9D | 9E | 9F |
|---|---|---|---|---|---|---|
| Dissolution rate after 4 hours (%) | 30 | 29 | 32 | 29 | 27 | 16 |
| Dissolution rate after 24 hours (%) | 85 | 87 | 87 | >80 | >79 | >61 |

### Example 10: Matrix formulation utilizing water-soluble polymer (HPMC matrix)

In accordance with the formulations shown in Table 13, 6 mg of solid dispersion 2 (corresponding to 1 mg of tacrolimus) which is prepared in a similar fashion as shown in Example 2 is mixed with hydroxypropylmethyl cellulose (HPMC), and compression-molded by using an oil press tabletting machine (tabletting pressure = 1000 kg/punch), to obtain sustained release tacrolimus formulations (10A to 10F) of the present invention.
As HPMC, TC5S (Shin-Etsu Chemical Co., Ltd.), 60SH50 (Shin-Etsu Chemical Co., Ltd.), and 60SH400 (Shin-Etsu Chemical Co., Ltd.) are used.

**[Table 13]**

| Components (unit:mg) | 10A | 10B | 10C |
|---|---|---|---|
| Solid dispersion 2 | 6 | 6 | 6 |
| HPMC (TC5S) | 395 | 345 | 295 |
| Total weight (mg) | 401 | 351 | 301 |
| Tablet size (mm) | 9.5×9.5R | 9×9R | 8.5×8.5R |
| | | | |

| Components (unit:mg) | 10D | 10E | 10F |
|---|---|---|---|
| Solid dispersion 2 | 6 | 6 | 6 |
| Mixture of lactose and magnesium stearate | 60 | 60 | 60 |
| HPMC (60SH50) | 250 | 200 | - |
| HPMC (60SH400) | - | - | 150 |
| Total weight (mg) | 316 | 266 | 216 |
| Tablet size (mm) | 9×9R | 8.5×8.5R | 8.5×8.5R |

### Example 11: Matrix formulation utilizing water-soluble polymer (HPMC + PVP matrix)

In accordance with the formulations shown in Table 14, 5 mg of solid dispersion 1 (corresponding to 1 mg of tacrolimus) prepared in a similar fashion as shown in Example 1 was mixed with polyvinylpyrrolidone (PVP) and hydroxypropylmethyl cellulose (HPMC), and compression-molded by using an oil press tabletting machine (tabletting pressure = 1000 kg/punch), to obtain sustained release tacrolimus formulations (11A to 11D) of the present invention.
K90 (Wako Pure Chemical Industries, Ltd.) was used as PVP, and 90SH100000 (Shin-Etsu Chemical Co., Ltd.) was used as HPMC.

**[Table 14]**

| Components (unit:mg) | 11A | 11B | 11C | 11D |
|---|---|---|---|---|
| Solid dispersion 1 | 5 | 5 | 5 | 5 |
| Mixture of lactose and magnesium stearate | 60 | 60 | 60 | 60 |
| PVP | 116 | 100 | 170 | 200 |
| HPMC | 84 | 100 | 100 | 200 |
| Total weight (mg) | 265 | 265 | 335 | 465 |
| Tablet size (mm) | 8.5×8.5R | 8.5×8.5R | 9×9R | 10.5×10.5R |

### Test Example 6: Dissolution test

Drug-releasing properties of formulations 11A to 11D prepared in Example 11 were evaluated by the method described in Test Example 3.

### Results:

Dissolution rates of formulations 11A to 11D after 4 hours and 24 hours from the beginning of the dissolution test are shown in Table 15. The dissolution rates of formulations 11A to 11D were 15% to 26% after 4 hours from the beginning of the dissolution test, and 75% or more after 24 hours therefrom.

**[Table 15]**

| Examples | 11A | 11B | 11C | 11D |
|---|---|---|---|---|
| Dissolution rate after 4 hours (%) | 26 | 20 | 26 | 15 |
| Dissolution rate after 24 hours (%) | 94 | 80 | 81 | 75 |

### Example 12: Matrix formulation utilizing water-soluble polymer (HPMC + PVP matrix)

In accordance with the formulations shown in Table 16, 6 mg of solid dispersion 2 (containing the water-insoluble base and the equivalent corresponding to 1 mg of tacrolimus) which is prepared in a similar fashion as shown in Example 2 is mixed with polyvinylpyrrolidone [PVP K90 (Wako Pure Chemical Industries, Ltd.)] and hydroxypropylmethyl cellulose [HPMC 90SH100000 (Shin-Etsu Chemical Co., Ltd.)], and compression-molded by using an oil press tabletting machine (tabletting pressure = 1000 kg/punch), to obtain sustained release tacrolimus formulations (12A to 12D) of the present invention.

**[Table 16]**

| Components (unit:mg) | 12A | 12B | 12C | 12D |
|---|---|---|---|---|
| Solid dispersion 2 | 6 | 6 | 6 | 6 |
| Mixture of lactose and magnesium stearate | 60 | 60 | 60 | 60 |
| PVP | 116 | 100 | 170 | 200 |
| HPMC | 84 | 100 | 100 | 200 |
| Total weight (mg) | 266 | 266 | 336 | 466 |
| Tablet size (mm) | 8.5×8.5R | 8.5×8.5R | 9×9R | 10.5×10.5R |

### Example 13: Formulation utilizing water-soluble polymer matrix (HPMC + PVA matrix)

In accordance with the formulations shown in Table 17, 5 mg of solid dispersion 1 (corresponding to 1 mg of tacrolimus) prepared in a similar fashion as shown in Example 1 was mixed with polyvinyl alcohol (PVA) and hydroxypropylmethyl cellulose (HPMC), and compression-molded by using an oil press tabletting machine (tabletting pressure = 1000 kg/punch), to obtain sustained release tacrolimus formulations (13A and 13B) of the present invention.
PVA having a molecular weight of 105,000 (Denki Kagaku Kogyo Kabushiki Kaisha) was used as PVA, and 90SH100000 (Shin-Etsu Chemical Co., Ltd.) was used as HPMC.

**[Table 17]**

| Components (unit:mg) | 13A | 13B |
|---|---|---|
| Solid dispersion 1 | 5 | 5 |
| Mixture of lactose and magnesium stearate | 60 | 60 |
| PVA | 75 | 150 |
| HPMC | 75 | 50 |
| Total weight (mg) | 215 | 265 |
| Tablet size (mm) | 8×8R | 8.5×8.5R |

### Test Example 7: Dissolution test

Drug-releasing properties of formulations 13A and 13B prepared in Example 13 were evaluated by the method described in Test Example 1.

### Results:

Dissolution rates of formulations 13A and 13B after 4 hours and 24 hours from the beginning of the dissolution test are shown in Table 18. The dissolution rates of formulations 13A and 13B were 12% and 14% after 4 hours from the beginning of the dissolution test, respectively, and 60% and 69% after 24 hours therefrom, respectively.

**[Table 18]**

| Examples | 13A | 13B |
|---|---|---|
| Dissolution rate after 4 hours (%) | 12 | 14 |
| Dissolution rate after 24 hours (%) | 60 | 69 |

### Example 14: Matrix formulation utilizing water-soluble polymer (HPMC + PVA matrix)

In accordance with the formulations shown in Table 19, 6 mg of solid dispersion 2 (containing the water-insoluble base and the equivalent corresponding to 1 mg of tacrolimus) which is prepared in a similar fashion as shown in Example 2 is mixed with polyvinyl alcohol [PVA, molecular weight: 105,000 (Denki Kagaku Kogyo Kabushiki Kaisha)] and hydroxypropylmethyl cellulose [HPMC, 90SH100000(Shin-Etsu Chemical Co., Ltd.)], and compression-molded by using an oil press tabletting machine (tabletting pressure = 1000 kg/punch), to obtain sustained release tacrolimus formulations (14A and 14B) of the present invention.

**[Table 19]**

| Components (unit:mg) | 14A | 14B |
|---|---|---|
| Solid dispersion 2 | 6 | 6 |
| Mixture of lactose and magnesium stearate | 60 | 60 |
| PVA | 75 | 150 |
| HPMC | 75 | 50 |
| Total weight (mg) | 216 | 266 |
| Tablet size (mm) | 8×8R | 8.5×8.5R |

### Example 15: Matrix formulation utilizing water-soluble polymer (PVA matrix)

In accordance with the formulations shown in Table 20, 5 mg of solid dispersion 1 (corresponding to 1 mg of tacrolimus) prepared in a similar fashion as shown in Example 1 was mixed with lactose and polyvinyl alcohol (PVA), and compression-molded by using an oil press tabletting machine (tabletting pressure = 1000 kg/punch), to obtain sustained release tacrolimus formulations (15A to 15C) of the present invention.
PVA having a molecular weight of 105,000 (Denki Kagaku Kogyo Kabushiki Kaisha) was used as PVA.

**[Table 20]**

| Components (unit:mg) | 15A | 15B | 15C |
|---|---|---|---|
| Solid dispersion 1 | 5 | 5 | 5 |
| lactose | - | 15 | 30 |
| PVA | 160 | 145 | 130 |
| Total weight (mg) | 165 | 165 | 165 |
| Tablet size (mm) | 7×7R | 7×7R | 7×7R |

### Test Example 8: Dissolution test

Drug-releasing properties of formulations 15A to 15C prepared in Example 15 were evaluated by the method described in Test Example 1.

### Results:

Dissolution rates of formulations 15A to 15C after 4 hours and 24 hours from the beginning of the dissolution test are shown in Table 21. The dissolution rates of formulations 15A to 15C were 8% to 14% after 4 hours from the beginning of the dissolution test, and 74% or more after 24 hours therefrom.

**[Table 21]**

| Examples | 15A | 15B | 15C |
|---|---|---|---|
| Dissolution rate after 4 hours (%) | 10 | 8 | 14 |
| Dissolution rate after 24 hours (%) | 82 | 74 | 90 |

### Example 16: Matrix formulation utilizing water-soluble polymer (PVA matrix)

In accordance with the formulations shown in Table 22, 6 mg of solid dispersion 2 (containing the water-insoluble base and the equivalent corresponding to 1 mg of tacrolimus) which is prepared in a similar fashion as shown in Example 2 is mixed with lactose and polyvinyl alcohol [PVA, molecular weight: 105,000 (Denki Kagaku Kogyo Kabushiki Kaisha)], and compression-molded by using an oil press tabletting machine (tabletting pressure = 1000 kg/punch), to obtain sustained release tacrolimus formulations (16A to 16C) of the present invention.

**[Table 22]**

| Components (unit:mg) | 16A | 16B | 16C |
|---|---|---|---|
| Solid dispersion 2 | 6 | 6 | 6 |
| Lactose | - | 15 | 30 |
| PVA | 160 | 145 | 130 |
| Total weight (mg) | 166 | 166 | 166 |
| Tablet size (mm) | 7×7R | 7×7R | 7×7R |

### Example 17: Osmotic pump type formulation

### Step 1: Preparation of mixed powder which forms drug layer containing active substance

In accordance with the formulations shown in Table 23, mixed powders D1 and D2 consisting of 5 mg of solid dispersion 1 (corresponding to 1 mg of tacrolimus) prepared in a similar fashion as shown in Example 1, and 6 mg of solid dispersion 2 (corresponding to 1 mg of tacrolimus) which is prepared in a similar fashion as shown in Example 2, respectively, as well as polyethylene oxide (Polyox WSR N80) and hydroxypropylmethyl cellulose (HPMC2910), are prepared and used in preparing a bilayered compressed core.

**[Table 23]**

| Components (unit:mg) | D1 | D2 |
|---|---|---|
| Solid dispersion 1 | 5 | |
| Solid dispersion 2 | | 6 |
| Polyox WSR N8 | 100 | 100 |
| HPMC 2910 | 6 | 6 |
| Magnesium stearate | 1 | 1 |
| Total | 112 | 113 |

### Step 2: Preparation of push layer

Mixed powder E of the composition shown in Table 24 is prepared and used in a bilayered compressed core.

**[Table 24]**

| Components (unit:mg) | E |
|---|---|
| Polyox WSR Coagulant | 60 |
| NaCl | 30 |
| HPMC2910 | 4 |
| Red ferric oxide | 1 |
| Magnesium stearate | 0.5 |
| Total | 95.5 |

### Step 3: Preparation of bilayered compressed core consisting of drug layer and push layer

A bilayered compressed core is prepared using an oil press tabletting machine. The tabletting is carried out using a die and punch having a diameter of 8.0 mm × 9.6R. The mixed powder E for a push layer is put into the die, and the mixed powder D1 or D2 for a drug layer is further loaded onto the push layer in the die. Compression-molding is carried out to obtain bilayered compressed cores containing 1 mg of tacrolimus.

### Step 4: Preparation of semipermeable membrane and membrane coating

PEG 4000 and cellulose acetate [94:6(W/W%)] are dissolved in a mixed solvent of dichloromethane and methanol [9:1(W/W%)] to prepare a membrane-coating solution. This coating solution contains approximately 4% of solids when used. Each bilayered compressed core prepared in Step 3 is spray-coated with this coating solution using an aeration type coater (HiCoater HCT-30, manufactured by Freund Corporation) until 10W/W% of the coating has been applied with respect to the weight of the bilayered compressed core.

### Step 5: Making orifices

A needle (27G) having a diameter of 0.4 mm is used to form orifices at the drug layer side of coated tablets prepared in Step 4, to prepare a sustained release tacrolimus formulation (17) of the present invention.

### Example 18: Sustained release formulation with coating membrane

In accordance with the formulations shown in Table 25, 5 mg of solid dispersion 1 (corresponding to 1 mg of tacrolimus) prepared in a similar fashion as shown in Example 1, or 6 mg of solid dispersion 2 (corresponding to 1 mg of tacrolimus) which is prepared in a similar fashion as shown in Example 2, is mixed with 60 mg of a mixture of lactose and magnesium stearate (St-Mg) to obtain each mixed powder. Each mixture is formed into tablets under a tabletting pressure of 40 kg/cm² using a punch having a diameter of 5 mm × 6 mmR, to obtain uncoated tablets. Next, 7 parts of Eudragit RS100 (degussa), 3 parts of Eudragit RL100 (degussa), and 4 parts of polyethylene glycol (PEG 400) are added to 50 parts of dichloromethane, and dissolved by stirring using a magnetic stirrer, to prepare a coating liquid. The obtained uncoated tablets are immersed in this coating liquid, to obtain sustained release tacrolimus formulations (18A and 18B) of the present invention in which 13.5 wt% of a coating membrane, with respect to the weight of the uncoated tablet, is formed.

**[Table 25]**

| Components | 18A | 18B |
|---|---|---|
| [Uncoated tablet](unit:mg) | | |
| Solid dispersion 1 | 5 | |
| Solid dispersion 2 | | 6 |
| Mixture of lactose and magnesium stearate | 60 | 60 |
| Total weight | 65 | 66 |
| Tablet size (mm) | 5×6R | 5×6R |
| [Coating liquid] | | |
| Eudragit RS100 | 1.4 g | 1.4 g |
| Eudragit RL100 | 0.6 g | 0.6 g |
| PEG 400 | 0.8 g | 0.8 g |
| Dichloromethane | 25 mL | 25 mL |
| Coating ratio | 13.5% | 13.5% |

### Experimental Example 1: Evaluation of influence of food intake on PK of tacrolimus in dogs

Formulation 1A prepared in Example 1 or formulation prepared in Comparative Example 1 was orally administered to dogs, and the influence of food intake on an oral absorption of tacrolimus was evaluated. An administration in a fasted state was carried out by administering a test formulation to a subject which had been fasted for 12 hours or more. An administration after eating a meal was carried out by administering a test formulation to a subject after 30 minutes from the intake of 50 g of a meat diet. The dogs were allowed to freely take water, and 20 mL of water was given when each formulation was orally administered. Blood samples were sequentially collected after the oral administration, and blood concentrations of tacrolimus in the blood samples were measured with LC-MS/MS. From the time course of obtained blood concentrations, maximum blood tacrolimus concentrations (Cmax) and areas under the blood tacrolimus concentration versus time curve (AUC) after administrations under various conditions were calculated. With respect to each formulation, averages of a ratio of Cmax or AUC when administered after eating a meal to that when administered in a fasted state are shown in Table 26.

**[Table 26]**

| Formulations | Ratio of Cmax after eating to that in fasted state | Ratio of AUC after eating to that in fasted state |
|---|---|---|
| Comparative Example 1* | 0.25 | 0.44 |
| Example 1A** | 1.07 | 1.08 |

| | | |
|---|---|---|
| *: Average (n=6), **: Average (n=5) | | |

In Comparative Example 1, the ratios of Cmax and AUC when administered after eating a meal to those when administered in a fasted state were 0.25 and 0.44, respectively, and the food intake significantly affected the dogs with statistically - significant difference (p<0.05). By contrast, with respect to formulation 1A prepared in Example 1, as an embodiment of the sustained release pharmaceutical composition for tacrolimus of the present invention, the ratios of Cmax and AUC when administered after eating a meal to those when administered in a fasted state were 1.07 and 1.08, respectively, and the food intake did not affect the dogs. Like in dogs, it is expected in humans that the influence of food intake observed in Comparative Example 1 is decreased in the sustained release pharmaceutical composition for tacrolimus of the present invention.

### Experimental Example 2: Evaluation of PK of tacrolimus in dogs

Formulation 1A prepared in Example 1 or formulation prepared in Comparative Example 1 was orally administered to dogs, and the time course of blood tacrolimus concentrations was evaluated. Each formulation was administered to dogs which had been fasted for 12 hours or more in a fasted state. The dogs were allowed to freely take water, and 20 mL of water was given when each formulation was orally administered. Blood samples were sequentially collected after the administration, and blood concentrations of tacrolimus in the blood samples were measured with LC-MS/MS. From the time course of obtained blood concentrations, maximum blood tacrolimus concentrations (Cmax) and blood tacrolimus concentrations after 8 hours from the administration (C8h) were calculated. With respect to each formulation, averages of a ratio of Cmax to C8h are shown in Table 27.

**[Table 27]**

| Formulations | Cmax/C8h |
|---|---|
| Comparative Example 1 | 10.1 |
| Example 1A | 2.6 |

| | |
|---|---|
| Average (n=5) | |

Whereas the ratio of Cmax to C8h in Comparative Example 1 was 10.1, the ratio in Example 1A was 2.6, which was approximately 1/4 in comparison with Comparative Example 1. There are apprehensions in Comparative Example 1 that a high Cmax enhances the risk of development of adverse effects, and that insufficient effects are maintained due to a low C8h. By contrast, in Example 1A, a small difference between Cmax and C8h and a constant blood concentration profile were observed, and therefore, it is expected that the risk of development of adverse effects is decreased, and that sufficient effects are maintained. Like in dogs, it is expected in humans that a peak/trough ratio of blood tacrolimus concentrations in Comparative Example 1 is decreased and thus safer effects are maintained in the sustained release pharmaceutical composition for tacrolimus of the present invention.

### INDUSTRIAL APPLICABILITY

According to the present invention, in which a dissolution rate of tacrolimus after 4 hours from the beginning of dissolution is less than 35%, when a sustained release pharmaceutical composition containing tacrolimus is orally administered, food effects can be avoided and the safety profile of tacrolimus can be improved. Therefore, it is expected that the present invention contributes to the improvement of QOL for patients and compliance.
As above, the present invention was explained with reference to particular embodiments, but modifications and improvements obvious to those skilled in the art are included in the scope of the present invention.

## Claims

1. A sustained release pharmaceutical composition for tacrolimus, comprising a solid dispersion containing tacrolimus or a pharmaceutically acceptable salt thereof, and a carrier for a sustained release pharmaceutical composition, wherein a dissolution rate of tacrolimus after 4 hours from the beginning of a dissolution test is less than 35%.

2. The sustained release pharmaceutical composition for tacrolimus according to claim 1, wherein a ratio of a maximum blood tacrolimus concentration (Cmax) when administered after eating a meal to a maximum blood tacrolimus concentration when administered in a fasted state is 0.3 or more, and/or a ratio of an area under a blood tacrolimus concentration versus time curve (AUC) when administered after eating a meal to an area under a blood tacrolimus concentration versus time curve when administered in a fasted state is 0.5 or more.

3. The sustained release pharmaceutical composition for tacrolimus according to claim 1, wherein a ratio of a maximum blood tacrolimus concentration (Cmax) when administered after eating a meal to a maximum blood tacrolimus concentration when administered in a fasted state is 0.7 or more, and/or a ratio of an area under a blood tacrolimus concentration versus time curve (AUC) when administered after eating a meal to an area under a blood tacrolimus concentration versus time curve when administered in a fasted state is 0.8 or more.

4. The sustained release pharmaceutical composition for tacrolimus according to claim 1, wherein a ratio of a maximum blood tacrolimus concentration (Cmax) to a blood tacrolimus concentration after approximately 8 hours from oral administration of tacrolimus (C8h) is 5 or less.

5. The sustained release pharmaceutical composition for tacrolimus according to claim 1, wherein a ratio of a maximum blood tacrolimus concentration (Cmax) to a blood tacrolimus concentration after approximately 24 hours from oral administration of tacrolimus (Cmin) is 3 or less.

6. The sustained release pharmaceutical composition for tacrolimus according to claim 1, which is selected from the group consisting of a hydrogel-forming formulation, an osmotic pump type formulation, a gel formulation in which a plurality of gums is combined, a multi-layered tablet formulation consisting of a drug core and a release-controlling layer which are geometrically arranged, a formulation utilizing a swelling polymer, a matrix formulation utilizing a water-soluble polymer, and a sustained release formulation with a coating membrane.

7. The sustained release pharmaceutical composition for tacrolimus according to claim 6, wherein the hydrogel-forming formulation comprises a hydrophilic base and a hydrogel-forming polymer.

8. Use of tacrolimus or a pharmaceutically acceptable salt thereof for the manufacture of a sustained release pharmaceutical composition for tacrolimus, wherein a dissolution rate of tacrolimus after 4 hours from the beginning of a dissolution test is less than 35%, and an effect of a meal on tacrolimus can be avoided.

9. Use of tacrolimus or a pharmaceutically acceptable salt thereof according to claim 8, wherein a ratio of a maximum blood tacrolimus concentration (Cmax) when administered after eating a meal to a maximum blood tacrolimus concentration when administered in a fasted state is 0.3 or more, and/or a ratio of an area under a blood tacrolimus concentration versus time curve (AUC) when administered after eating a meal to an area under a blood tacrolimus concentration versus time curve when administered in a fasted state is 0.5 or more.

10. Use of tacrolimus or a pharmaceutically acceptable salt thereof according to claim 8, wherein a ratio of a maximum blood tacrolimus concentration (Cmax) when administered after eating a meal to a maximum blood tacrolimus concentration when administered in a fasted state is 0.7 or more, and/or a ratio of an area under a blood tacrolimus concentration versus time curve (AUC) when administered after eating a meal to an area under a blood tacrolimus concentration versus time curve when administered in a fasted state is 0.8 or more.

11. Use of tacrolimus or a pharmaceutically acceptable salt thereof according to claim 8, wherein the sustained release pharmaceutical composition for tacrolimus comprises a hydrophilic base and a hydrogel-forming polymer.

12. Use of tacrolimus or a pharmaceutically acceptable salt thereof for the manufacture of a sustained release pharmaceutical composition for tacrolimus, wherein a dissolution rate of tacrolimus after 4 hours from the beginning of a dissolution test is less than 35%, and the safety profile of tacrolimus can be improved.

13. Use of tacrolimus or a pharmaceutically acceptable salt thereof according to claim 12, wherein a ratio of a maximum blood tacrolimus concentration (Cmax) to a blood tacrolimus concentration after approximately 8 hours from oral administration of tacrolimus (C8h) is 5 or less.

14. Use of tacrolimus or a pharmaceutically acceptable salt thereof according to claim 12, wherein a ratio of a maximum blood tacrolimus concentration (Cmax) to a blood tacrolimus concentration after approximately 24 hours from oral administration of tacrolimus (Cmin) is 3 or less.

15. Use of tacrolimus or a pharmaceutically acceptable salt thereof according to claim 12, wherein the sustained release pharmaceutical composition for tacrolimus comprises a hydrophilic base and a hydrogel-forming polymer.

16. A method of regulating a ratio of a maximum blood tacrolimus concentration (Cmax) when administered after eating a meal to a maximum blood tacrolimus concentration when administered in a fasted state to 0.3 or more, and/or a ratio of an area under a blood tacrolimus concentration versus time curve (AUC) when administered after eating a meal to an area under a blood tacrolimus concentration versus time curve when administered in a fasted state to 0.5 or more, by dissolving tacrolimus contained in a sustained release pharmaceutical composition at a dissolution rate of tacrolimus after 4 hours from the beginning of a dissolution test of less than 35%.

17. A method of regulating a ratio of a maximum blood tacrolimus concentration (Cmax) when administered after eating a meal to a maximum blood tacrolimus concentration when administered in a fasted state to 0.7 or more, and/or a ratio of an area under a blood tacrolimus concentration versus time curve (AUC) when administered after eating a meal to an area under a blood tacrolimus concentration versus time curve when administered in a fasted state to 0.8 or more, by dissolving tacrolimus contained in a sustained release pharmaceutical composition at a dissolution rate of tacrolimus after 4 hours from the beginning of a dissolution test of less than 35%.

18. A method of regulating a ratio of a maximum blood tacrolimus concentration (Cmax) to a blood tacrolimus concentration after approximately 8 hours from oral administration of tacrolimus (C8h) to 5 or less, by dissolving tacrolimus contained in a sustained release pharmaceutical composition at a dissolution rate of tacrolimus after 4 hours from the beginning of a dissolution test of less than 35%.

19. A method of regulating a ratio of a maximum blood tacrolimus concentration (Cmax) to a blood tacrolimus concentration after approximately 24 hours from oral administration of tacrolimus (Cmin) to 3 or less, by dissolving tacrolimus contained in a sustained release pharmaceutical composition at a dissolution rate of tacrolimus after 4 hours from the beginning of a dissolution test of less than 35%.
